# EUROPÄISCHE PATENTSCHRIFT

(11) **EP 2 264 069 B1**
(45) Veröffentlichungstag und Bekanntmachung des Hinweises auf die Patenterteilung: **17.04.2013**
(21) Anmeldenummer: 10182320.1
(22) Anmeldetag: 12.02.2005
(51) Int. Cl.: C07K 14/75

(54) **Reinigung von Fibrinogen**
Purification of fibrinogen
Purification du fibrinogène

(30) Priorität: 24.02.2004 DE 102004009400
(43) Veröffentlichungstag der Anmeldung: 22.12.2010
(62) Teilanmeldung aus: 05003017.0
(73) Patentinhaber: CSL Behring GmbH, 35041 Marburg (DE)
(72) Erfinder: Feussner, Annette, 35043 Marburg (DE); Gawantka, Volker, 35094 Lahntal (DE); Lemmer, Jörg, 35085 Ebsdorfergrund (DE); Liebing, Uwe, 35091 Coelbe (DE); Metzner, Hubert, 35041 Marburg (DE); Schulte, Stefan, 35043 Marburg (DE)
(74) Vertreter: Binsack, Beate

(56) Entgegenhaltungen:
- EP-A- 0 311 950
- EP-A2- 0 190 606
- WO-A-00/17234
- WO-A-93/05067
- DE-A1- 2 903 131
- US-A- 4 096 136
- US-A- 6 037 457

## Beschreibung

Die vorliegende Erfindung betrifft ein Verfahren zur Reinigung von Fibrinogen, **dadurch gekennzeichnet, dass** es einen oder mehrere Verfahrensschritte enthält, bei denen ein oder mehrere kontaminierende Proteine durch negative Chromatographie und/oder negative Adsorption, unter Verwendung von Farbstoffgel abgereichert werden.

Fibrinogen nimmt eine Schlüsselrolle bei der Blutgerinnung ein. Bei Verletzungen oder Operationen werden fast immer Blutgefäße beschädigt und es entstehen Blutungen. Durch die Blutgerinnung wird das Blut im Bereich kleinerer Wunden verfestigt und die Blutung kommt zum Stillstand. Das Gerinnungssystem schützt den Körper damit vor hoher Blutverlusten. Bei der Blutgerinnung wird das im Blutplasma enthaltene lösliche Fibrinogen in Anwesenheit von Thrombin in das faserartige, unlösliche Fibrin umgewandelt. Fehlt es an Fibrinogen, so funktioniert die Blutgerinnung nicht korrekt. Man kann den Mangel ausgleichen, indem man Fibrinogen, isoliert aus z.B. menschlichem Blutplasma, verabreicht. Wegen seiner Bedeutung für die Blutstillung und Wundheilung hat Fibrinogen eine hohe Bedeutung in der klinischen Anwendung.

Aufgrund dieser hohen klinischen Bedeutung von Fibrinogen gibt es in der Literatur vielfältige Hinweise, die sich mit verschiedenen Methoden für die Reinigung dieses wichtigen Proteins beschäftigen. Die Reinigung von Fibrinogen erfolgt vorwiegend aus humanem, seltener auch aus tierischem Plasma. Ebenfalls möglich ist eine Reinigung von rekombinantem Fibrinogen, z. B. aus der Zellkultur nach rekombinanter Expression, oder auch aus der Milch transgener Tiere.

Das humane Plasma enthält eine komplexe Mischung von mehr als 100 Proteinen, wobei Fibrinogen etwa 2% der gesamten Proteinmenge ausmacht. Die Reinigung und Isolierung von Fibrinogen benötigt daher im Regelfall mehrere Schritte und die Kombinationsmöglichkeiten dieser einzelnen Verfahrenssschritte sind vielfältig.

Ein wichtiger Bestandteil der Reinigung von Fibrinogen aus humanem Plasma ist traditionell die Präzipitation. Bekannte Präzipitationsmethoden verwenden Aminosäuren wie Glycin oder Alanin, siehe beispielsweise die EP 0 383 234, die WO 01/48016 oder Jakobsen & Kierulf (Thrombosis Research 3 (1973) 145-159), Ammoniumsulfat, siehe beispielsweise die US 5,773,033, US 6,037,457 oder Takeda (Journal of Clinical Investigation 45 (1966) 103-111), Polymere wie beispielsweise Polyethylenglykol (PEG), siehe beispielsweise die WO 95/25748 oder Vila et al. (Thrombosis Research 39 (1985) 651-656), Ethanol, siehe beispielsweise EP 0 408 029, wo Fibrinogen mit 5-10% Ethanol ausgefällt und von anderen Plasmaproteinen getrennt wird, oder die US 5,099,003 oder Blombäck & Blombäck (Arkiv För Kemi 10 (1956) 415-443), Sulfatierte Polysaccharide (SPS, z.B. Heparin), siehe beispielsweise WO 99/37680 und US 4,210,580 und Lösungen geringer lonenstärke, siehe beispielsweise US 4,188,318 und DE 26 36 757.

Die Reinheit von allein auf Grundlage von Präzipitationen gewonnenem Fibrinogen ist für einige Anwendungen jedoch noch nicht ausreichend, so dass im Stand der Technik zusätzlich oder alternativ verschiedene Adsorptions- und Chromatographieschritte zur Aufreinigung von Fibrinogen beschrieben sind.

Im Bereich der lonenaustauschchromatographie wird häufig die Anionenaustauschchromatographie verwendet. Verwiesen sei in diesem Zusammenhang auf die EP 0 555 135, in der Fibrinogen in einem Hauptverfahrensschritt an eine Anionenaustauscher Säule gebunden wird, während z.B. Albumin und Inaktivatoren nicht binden. Fibrinogen wird nachfolgend von der Säule eluiert. In der WO 93/05067 wird das Binden von Fibrinogen an Anionenaustauscher genutzt, um für die Virusinaktivierung zugesetzte Detergenzien wieder zu entfernen. Die WO 01/48016 beschreibt die Bindung von Fibrinogen an lonenaustauschmaterial unter vorzugsweiser Verwendung der den Fibrinogenabbau verzögernden ω-Aminosäuren, wie beispielsweise die ε -Aminocapronsäure (EACA) im Auftrags- und/oder Waschpuffer. Ein solcher Verfahrensschritt ermöglicht insbesondere eine effiziente Abreicherung des kontaminierenden Plasminogens aus fibrinogenhaltigen Lösungen.

Die Kationenaustauscher Chromatographie findet Anwendung beispielsweise in der Aufreinigung von Fibrinogen aus der Milch transgener Tiere beschrieben in der WO 00/17234. Auch in diesem Fall werden Bedingungen gewählt, die die Bindung von Fibrinogen an das Säulenmaterial zur Folge haben und damit die Abtrennung von z.B. Casein ermöglichen.

Die Eigenschaft des Bindens von Fibrinogen an Kationenaustauscher wird in der WO 91/01808 ausgenutzt, um selektiv Fibrinogen, Lipoproteine und Harnstoff aus Flüssigkeiten wie z. B. Blut zu entfernen.

In der WO 89/12065 wird Fibrinogen in einem Verfahrensschritt durch eine Heparin-Sepharose Säule aufgereinigt. Die Bedingungen sind so gewählt, dass Fibrinogen an das Säulenmaterial adsorbiert und so vor allem Albumine, Immunoglobuline und Virusinaktivierungs-Substanzen abgetrennt werden können.

Beschrieben ist auch die Möglichkeit, Fibrinogen über hydrophobe Wechselwirkungen aufzureinigen. In der WO 00/17239 wird die Reinigung von Fibrinogen aus Milch transgener Tiere in einem Verfahrensschritt durch Bindung an eine hydrophobe Säule in Anwesenheit von Salzen mit anschließender Elution erreicht. Die Möglichkeit der Anwendung einer hydrophoben Säule für Fibrinogen aus humanem Plasma wird ebenfalls erwähnt.
Beschrieben sind auch verschiedene Affinitätschromatographien, die über unterschiedliche Liganden verfügen, die selektiv Fibrinogen zu binden vermögen. Hingewiesen sei in diesem Zusammenhang z.B. auf die US 6,037,457 oder die WO 99/05176, die Antikörper beschreiben, die spezifisch Fibrinogen oder Fibrinogenpeptide binden und u.a. zur Reinigung von Fibrinogen eingesetzt werden können. Die EP 0 789 030, US 5,723,579 und US 5,783,663 beschreiben Peptide, die Fibrinogen binden und entsprechend als Liganden in Affinitätssäulen zur Reinigung von Fibrinogen verwendet werden können. Immobilisiertes Ristocetin wurde ebenfalls zum Binden von Fibrinogen eingesetzt und dieses mit 8 M Harnstoff wieder eluiert (Suzuki et al., Thrombosis Research 18 (1980) 707-715). In der WO 99/20655 schließlich wird inaktiviertes Thrombin als Ligand für Thrombin Substrate, zu denen auch Fibrinogen zählt, verwendet. In der WO 90/12803 wird die so genannte IMAC (Immobilized Metal Affinity Chromatography) zur Aufreinigung bestimmter Proteine beschrieben. Auch humanes Fibrinogen ist als mögliches Protein aufgeführt, das an die Metallchelat-Matrix bindet. Fibrinogen bindet auch an Protamin-Agarose und kann anschließend im sauren pH eluiert werden, siehe beispielsweise die US 6,037,457 oder Dempfle & Heene (Thrombosis Research 46 (1987) 19-27).

Allen diesen aufgeführten Verfahren oder Verfahrensschritten ist gemeinsam, dass die Bedingungen so gewählt sind, dass Fibrinogen an das Chromatographie- bzw. Adsorptionsmaterial bindet und anschließend wieder von dem Gelmaterial abgelöst werden muss.

Verfahren, bei denen Fibrinogen die Säule passiert, sind bisher lediglich für Verfahren beschrieben, bei denen die Auftrennung des Ausgangsmaterials in verschiedene "Nutzfraktionen" gewünscht ist oder bei denen Fibrinogen eine Verunreinigung ist.

Plasma oder Kryopräzipitat enthält neben Fibrinogen auch noch große Mengen weiterer klinisch bedeutungsvoller Plasmaproteine, so dass es auch als Ausgangsmaterial zur Fraktionierung und Gewinnung von Faktor VIII (F VIII), Fibronektin und von Willebrandfaktor verwendet wird. Aus diesem Grund werden auch chromatographische Verfahrensschritte zur Anwendung gebracht, die alle vier oder zumindest zwei der Hauptproteine zu trennen vermögen. Hierzu werden Anionenaustauscher unter Bedingungen verwendet, die Fibrinogen nicht binden, beispielsweise aber F VIII, von Willebrandfaktor und Fibronektin, die anschließend selektiv durch unterschiedliche lonenstärken eluiert werden. Verwiesen sei hierzu beispielsweise auf die WO 89/12065, die EP 0 359 593 oder die US 5,252,709. In der EP 0 383 234 werden für den Anionenaustauscher Bedingungen gewählt, bei denen nur F VIII bindet, von Willebrandfaktor, Fibronektin und Fibrinogen dagegen passieren die Säule bzw. bleiben im Überstand (Batch-Verfahren). Die EP 0 408 029 und US 5,138,034 schließlich beinhalten u.a. ein Fraktionierungsverfahren, bei dem durch eine Einfrier/Auftau-Behandlung zunächst F VIII und Fibonektin durch Präzipitation abgetrennt werden, Faktor IX (F IX) an eine sich anschließende Anionenaustauschersäule adsorbiert und das Fibrinogen aus dem Durchlauf der Säule über Ethanol ausgefällt wird. In allen diesen Fällen geht es um die Separation der klinisch bedeutungsvollen Hauptproteine, die dann vor ihrer pharmazeutischen Verwendung einzeln weiteren Reinigungsschritten zugeführt werden können. Verwendung findet ein ähnlicher Verfahrensschritt mit einer Anionenaustauschersäule auch als möglicher Zwischenschritt bei der Fibrinogenreinigung zur Entfernung von F VIII in der EP 0 555 135. In der WO 96/02571 bzw. US 5,834,420 wird in einer Ausführungsform zur Reinigung von Fibrinogen, lonenaustauschermaterial, ähnlich wie in der WO 89/12065, zur Entfernung von F VIII und von Willebrandfaktor eingesetzt. Proteine wie F VIII und von Willebrandfaktor haben allerdings keinen Einfluß auf die Stabilität von Fibrinogen. Häufig wird dieser Verfahrensschritt auch bei der Herstellung von F VIII Konzentraten angewendet, um diese möglichst vollständig von Kontaminanten, wie z.B. Immunglobulinen, Fibronektin und Fibrinogen, zu befreien. Verwiesen sei in diesem Zusammenhang beispielsweise auf die US 5,043,428 und EP 0 173 242. In der US 4,210,580 wird ein Verfahren zur Aufreinigung von Fibronektin beschrieben, bei dem Fibrinogen nach der Heparinpräzipitation, durch Waschen einer anschließenden Anionenaustauschersäule abgetrennt werden kann. Auch die US 5,099,003 nutzt einen Anionenaustauscher, in diesem Fall aber zur Entfernung der Faktoren II, VII, IX und X, die ansonsten nach einer Virus-Inaktivierungs Behandlung mit β-Propiolacton und UV-Bestrahlung, ein Gelieren und Klumpen der fibrinogenhaltigen Lösung verursachen würden. In der DE 29 02 158, die Verfahren zur Herstellung von Fibrinogen, Prothrombinkomplex, Antithrombin III und eine Lösung lagerstabiler Serumproteine beschreibt, wird u.a. eine Variante beschrieben, bei der zunächst der Prothrombinkomplex (F II, F VII, F IX und F X) durch Adsorption an Anionenaustauschern gewonnen wird und anschließend aus dem Durchlauf noch Fibrinogen durch Adsorption mit kolloidaler Kieselsäure isoliert wird. Da es bei dieser Reinigungsabfolge aber häufig bereits zu einer Fibrinolyse, die sich nachteilig auf die Fibrinogengewinnung auswirkt, kommt, wird die Variante bevorzugt, bei der zuerst Fibrinogen durch Adsorption an Kieselsäure abgetrennt und anschließend der Prothrombinkomplex mit Hilfe von Anionenaustauschern isoliert wird. In diesem Fall wirkt sich die Verwendung eines Anionenaustauschers also eher nachteilig auf die Qualität des gleichzeitig aus dem Durchlauf zu gewinnenden Fibrinogens aus.

In der WO 99/51724 schließlich wird ein "negatives" Chromatographieverfahren beschrieben, zur Aufreinigung heterologer Proteine, u.a. auch Fibrinogen, aus der Milch transgener Tiere. In diesem Fall wird jedoch Hydroxylapatit als Gelmaterial verwendet, um kontaminierende Milchproteine zu binden.

Auch beschrieben sind spezielle negative Affinitätschromatographien, wie beispielsweise immobilisierte Gelatine, die durch spezifische Bindung Fibronektin abzureichern vermag (siehe z.B. Vuento & Vaheri, Biochem. J. (1979) 183, 331-337). Fibronektin ist jedoch ein Strukturprotein, das keinen Einfluss auf die Stabilität von Fibrinogen hat.

Beschrieben ist auch, dass Lysin oder analoge Verbindungen sehr spezifisch Plasminogen zu binden vermag und diese Fähigkeit über eine Affinitätschromatographie mit L-Lysin substituierter Sepharose zur Reinigung von Plasminogen ausgenutzt werden kann (Deutsch & Mertz, Science 170 (1970) 1095-1096; Matsuda et al., Thrombosis Research 1 (1972) 619-630). Diese Fähigkeit von Lysin-Liganden wird in einigen Fibrinogen-Reinigungsverfahren als Verfahrensschritt ausgenutzt, um kontaminierendes Plasminogen abzureichern. Verwiesen sei diesbezüglich z.B. auf die WO 95/25748 oder WO 93/05067. In diesem Fall wird jedoch sehr spezifisch nur ein Protein, das Plasminogen, abgereichert, da das Lysin sehr spezifisch an ein bestimmtes Epitop (Kringel-Domänen) des Plasminogens bindet.

In der WO 01/27623 ist ein Verfahren beschrieben, wie Isoagglutinine (Blutgruppen-Antikörper) aus Blut oder Blutbestandteilen wie z.B. Fibrinogen, entfernt werden können. In diesem Fall werden Antigene (z.B. Oligosaccharide) als Liganden einer Affinitätssäule verwendet, die spezifisch Isoagglutinine binden können. Eine Entfernung von Isoagglutininen ist vorteilhaft zur Vermeidung von Komplikationen, wenn die Blutgruppen des Spenderblutes oder der Spenderblutkomponenten von der Blutgruppe des Empfängers abweichen. Isoagglutinine haben keinen Einfluß auf die Stabilität von Fibrinogen gegenüber Proteolyse.

Anwendung finden auch die EP 0 366 946 bzw. US 5,094,960, die ein Verfahren zur Entfernung lipidlöslicher Verfahrenschemikalien aus biologischem Material, wie beispielsweise Plasma, Kryopräzipitat, Gerinnungsfaktoren und Fibrinogen, beschreiben. Dabei wird eine hydrophobe Chromatographie-Säule verwendet, die zwar die Verfahrenschemikalien, nicht aber die Proteine des biologischen Materials unter den gewählten Bedingungen adsorbiert. In diesem Verfahren geht es lediglich um die Entfernung von chemischen Substanzen, die z.B. zur Inaktivierung von Viren zugesetzt wurden. Proteine, wie beispielsweise Gerinnungsfaktoren, zu denen u.a. auch fibrinogenabbauende Faktoren wie Faktor XI (F XI) gehören (Scott et al., Arch Biochem Biophys 249 (1986) 480-488), sollen unter den gewählten Bedingungen nicht binden und sollen in diesem Patent nicht abgereichert werden. Ähnlich sollen in der DE 29 03 131 lediglich Ionen durch die Verwendung von lonenaustauschermaterial ausgewechselt werden. In diesem Patent wird ein Verfahren beschrieben zur gleichzeitigen Reinigung von vier Produkten (Antihämophiles Globulin A, Prothrombinkomplex, Lösung lagerstabiler Serumproteine und Fibrinogen), wobei als Ausgangsmaterial ein Plasma benötigt wird, aus dem Calcium-lonen entfernt wurden bzw. bei Verwendung von Citratplasma zusätzlich Citrationen entfernt wurden. Kationenaustauscher ersetzen dabei die Calcium-lonen gegen Natrium- oder Wasserstoffionen und Anionenaustauscher die Citrationen gegen Chloridoder Hydroxidionen. Aus dem so erhaltenen lonenaustauscherplasma kann dann u.a. Fibrinogen durch Adsorption an Kieselsäure isoliert werden. Der in diesem Fall verwendete Kationenaustauscher dient aber lediglich einem Austausch der Calciumionen um das Einsetzen einer Gerinnungsreaktion zu vermeiden, eine Reinigung erfolgt durch diesen Schritt nicht.

Adsorptionen werden ebenfalls bei der Reinigung von Plasmaproteinen eingesetzt. So wird in der DE 29 03 131 eine Tricalciumphosphat (TCP) Adsorption zur Reinigung des Prothrombinkomplexes genutzt. In der WO 95/25748 wird es zur Entfernung des Prothombinkomplexes als ein Verfahrensschritt zur Aufreinigung von Fibrinogen eingesetzt. Zu nennen wären auch die Adsorption unter Verwendung von Aluminiumhydroxid (Al(OH)₃), das Faktoren des Prothrombinkomplexes bindet und z.B. auch bei der Entfernung von Lipiden eine Rolle spielen kann. Verwendung findet Al(OH)₃ beispielsweise in der EP 0 383 234 oder WO 01/48016. In diesem Zusammenhang zu nennen wäre auch eine mögliche Verwendung von Bariumsulfat (BaSO₄).

All den beschriebenen Verfahren zur negativen Chromatographie/Adsorption ist, mit Ausnahme der EP 0 366 946 bzw. US 5,094,960, in der nur Verfahrenschemikalien entfernt werden, sowie der DE 29 03 131, in der nur Calcium- und Citrationen entfernt werden, gemeinsam, dass andere Gelmaterialien verwendet werden, als sie für den (die) erfindungsgemäßen Verfahrensschritt(e) beschrieben sind. Hinzu kommt, dass der besondere Vorteil des erfindungsgemäßen Verfahrensschrittes darin liegt, dass durch effiziente Abreicherung von ein oder mehreren kontaminierenden Proteinen, insbesondere fibrinogenabbauenden Proteinen oder deren Vorläufer, ein Fibrinogenpräparat erhalten werden kann, dessen Stabilität in Lösung signifikant erhöht ist. Für die technische Gewinnung ist es von besonderem Interesse, ein Reinigungsverfahren zu entwickeln, das nicht nur unter großtechnischen Gesichtspunkten wirtschaftlich durchführbar ist, sondern auch zu einem Fibrinogen führt, das bei längerer Lagerung in Lösung weitestgehend stabil bleibt.

Bei Fibrinogen handelt es sich um ein sehr großes Protein mit einer komplexen Struktur. Es ist ein aus zwei symmetrischen Hälften bestehendes Glykoprotein von ca. 340 kDa. Je zwei alpha (Aα), beta (Bβ) und gamma (γ)-Ketten bilden eine längliche Molekülstruktur (ca. 47 nm), die drei Domänen bildet (eine zentrale E Domäne und zwei identische D Domänen). Diese komplexe Struktur ist unverzichtbar für die effiziente Bildung einer stabilen Fibrinmatrix. Insbesondere die alpha Kette ist von einem beginnenden proteolytischen Abbau durch fibrinogenabbauende Proteasen betroffen. Solche Schädigungen an der alpha Kette bewirken, nach Einwirkung von Thrombin, einen verzögerten Beginn der Gerinnung und lassen darauf schließen, dass eine Proteolyse der alpha Kette(n) die Fibrinpolymerisierung beeinflussen kann. Auch die dreidimensionale Struktur des Fibringerinnsels wird durch eine geschädigte alpha Kette beeinflusst. Es kommt zu einem feineren Fibrinnetzwerk mit dünneren Fibrillen und einer geringeren mechanischen Stabilität. Die C-terminalen Enden der alpha Ketten enthalten Aminosäuresequenzen, an denen Bindungen zwischen benachbarten Fibrinmolekülen, katalysiert durch aktivierten FXIII, aufgebaut werden. Fehlende Quervernetzungen verringern die Stabilität der Fibrinmatrix. Dies alles zeigt, dass Fibrinogen mit stark geschädigten alpha Ketten eine verminderte Qualität aufweist und im pharmazeutischen Einsatz z.B. bei Fibrinklebern nicht wünschenswert ist. Vergleichbares gilt auch für den proteolytischen Abbau der weiteren Fibrinogenketten, der in der Regel aber mit einer langsamereren Kinetik erfolgt. Der Vorteil der erfindungsgemäßen Verfahrensschritte liegt in der Abbreicherung von fibrinogenabbauenden Proteinen mit dem Resultat einer erhöhten Stabilität des Fibrinogens in wässriger Lösung.

Verfahren, die eine möglichst weitreichende Entfernung von fibrinogenabbauenden Proteinen bzw. deren Proenzymen bewirken sind also besonders vorteilhaft, da die Stabilität und Effizienz der erhaltenen Fibrinogenlösung auch bei längerer Lagerung in flüssiger Form entscheidend verbessert ist. Eine Aufbewahrung in flüssiger Form ist für Fibrinogen von besonderem Vorteil, da die sofortige Einsatzmöglichkeit des Wirkstoffes am Patienten möglich ist und sich somit der zeitliche Aufwand der Rekonstitution lyophilisierter Zubereitungen bzw. das Auftauen und Erwärmen eingefrorener Zubereitungen erübrigt. Aber auch bei einer Lagerung als Lyophilisat oder im gefrorenen Zustand ist es von Vorteil, wenn das rekonstituierte bzw. aufgetaute Fibrinogen noch länger stabil ist. Dies zeigt sich beispielsweise in Situationen, wo z. B. für Operationen vorsorglich Material rekonstituiert wurde, dann aber aus medizinischen Erwägungen heraus der Einsatz nicht notwendig wurde. Dieses Material muss bei nur kurzfristiger Stabilität verworfen werden und könnte nicht mehr zu einem späteren Zeitpunkt zum Einsatz kommen. Bei Verwendung von Fibrinklebern ist es insbesondere von Vorteil, wenn Fibrinogen in flüssiger Form vorliegt. Bei den kommerziell erhältlichen Klebern handelt es sich zumeist um zwei Komponenten. Die eine Komponente umfasst Fibrinogen, häufig zusammen mit Faktor XIII und einem Fibrinolyseinhibitor wie Aprotinin, und die andere Komponente umfasst Thrombin, häufig zusammen mit Calciumionen. Ein Rekonstituieren, um den Kleber einsatzbereit zu machen, benötigt relativ viel Zeit, zumal Fibrinogen in hohen Konzentrationen vorliegt.

Ein weiterer großer Vorteil des Fibrinogens, das nach dem erfindungsgemäßen Verfahren gewonnen wurde, ist die Möglichkeit einer Flüssiglagerung für eine bestimmte Zeit auch bei Raumtemperatur, womit sich z.B. die Anwendungseigenschaften in Notfallsituationen verbessern können. Auch auf längeren Versandwegen, wo tiefe Temperaturen ggfs. nicht durchgehend gewährleistet werden können, ist es von Vorteil, wenn die Stabilität auch bei Raumtemperatur während dieser Phase zu gewährleisten ist. Stabile Lagerung von Fibrinogen in Lösung erleichtert also in vielerlei Hinsicht die Herstellung, den Gebrauch, Transport und die Verabreichung am Patienten. Aufgrund der vorteilhaft erhöhten Stabilität des Fibrinogens, das nach dem erfindungsgemäßen Verfahren hergestellt wird, ist es zudem möglich, in vielen pharmazeutischen Zubereitungen auf den Zusatz von Fibrinolyseoder Fibrinogenolyseinhibitoren, die unter Umständen zu unerwünschten Nebenwirkungen führen können oder die zur Reduktion potentieller Risiken vermieden werden sollen, zu verzichten.

Zusammenfassend bleibt also trotz beschriebener zahlreicher Reinigungsmethoden weiterhin ein Bedarf an verbesserten Methoden, die eine wirtschaftliche, großtechnische Herstellung von stabilen Fibrinogenlösungen ermöglichen.

Der vorliegenden Erfindung liegt damit die Aufgabe zugrunde, ein Verfahren zur Reinigung von Fibrinogen anzugeben, das ein Fibrinogen hoher Stabilität bei guter Ausbeute bereitstellt. Überraschenderweise wurden Reinigungsverfahren gefunden, die auch im großtechnischen Maßstab wirtschaftlich durchführbar sind und zu einem Fibrinogen führen, dessen Stabilität in Lösung signifikant erhöht ist.

Gegenstand der Erfindung ist daher ein Verfahren zur Reinigung von Fibrinogen, **dadurch gekennzeichnet, dass** es einen oder mehrere Verfahrensschritte enthält, bei denen ein oder mehrere kontaminierende Proteine durch negative Chromatographie(n) und/oder negative Adsorption(en) unter Verwendung von Farbstoffgel abgereichert werden.

Die in dieser Erfindung vorgeschlagenen unterschiedlichen Farbstoffgele, die verschiedene Farbstoff-Liganden tragen, sind in der Lage, unterschiedliche Proteine abzureichern, was die Herstellung einer fibrinogenhaltigen Lösung mit hoher Stabilität des Fibrinogens ermöglicht.

Die Verwendung von anorganischen, schwerlöslichen Salzen wie TCP, Ba(SO₄) und Al(OH)₃ führt jedoch nicht zu einer erfindungsgemäßen Fibrinogenpräparation. Allerdings können die erfindungsgemäßen Gelmaterialen auch in Kombination mit anorganischen, schwerlöslichen Salzen wie TCP, Ba(SO₄) und Al(OH)₃ eingesetzt werden.

Als Maß für die Fibrinogen-Stabilität wird der Anteil an niedermolekularen Fibrinogen-Abbaufragmenten bei einer Lagerung bei 30°C betrachtet. Diese Abbaufragmente werden durch Größenausschlußchromatographie als Peaks mit einem kleineren Molekulargewicht als der Fibrinogen-Haupt-Peak bestimmt (Peak 4 oder ≤ Peak 4). Als stabil im Sinne der Erfindung gilt eine Fibrinogenpräparation dann, wenn der absolute Anteil an Fibrinogen-Abbaufragmenten (Peak 4 oder ≤ Peak 4) bei einer Lagerung bei 30°C nach einem Monat im flüssigen Zustand unterhalb von 3 %, bevorzugterweise unterhalb von 2,5 % liegt.

Reinigung im Sinne der vorliegenden Erfindung kann ein Chromatographisches Reinigungsverfahren oder ein Batch-Adsorptionsverfahren sein.

Bewährt haben sich Verfahren, bei denen zunächst durch einen oder mehrere Fällungsschritte eine Fibrinogenpräparation von mindestens 50% (w/w), bevorzugterweise mindestens 70 % (w/w), Reinheit erzeugt wird, die dann durch negative Chromatographie(n) und/oder negative Adsorption(en) unter Verwendung von Farbstoffgel und gegebenenfalls durch zusätzliche, beschriebene Verfahren weiter gereinigt wird.

Die Verfahren nach dem Stand der Technik unterscheiden sich grundsätzlich von dem erfindungsgemäßen Verfahrensschritt der negativen Chromatographie oder negativen Adsorption, bei dem die Bedingungen so gewählt werden, dass Fibrinogen nicht oder nur zu einem kleineren Anteil bindet und somit bei chromatographischer Trennung die Säule passiert. Der überwiegende Anteil an Fibrinogen befindet sich also im Durchlauf bzw. bei der Adsorption im Überstand. Der Vorteil des erfindungsgemäßen Verfahrensschrittes liegt u.a. darin, dass Bedingungen gewählt werden können, die Struktur und Funktionalität des Fibrinogen nicht beeinträchtigen und darüber hinaus zu hohen Ausbeuten führen. Da bei dem erfindungsgemäßen Verfahrensschritt, insbesonders nach vorheriger Reinigung durch Präzipitationen, im wesentlichen nur noch Nebenkomponenten gebunden und abgetrennt werden müssen, ist, bedingt durch kleine Säulendimensionen und eine einfache technische Ausrüstung, eine kostengünstige großtechnische Durchführung möglich. Demgegenüber beinhalten die bislang beschriebenen Chromatographien/Adsorptionen mit Bindung von Fibrinogen einige Nachteile. Da Fibrinogen gebunden wird, werden große Säulendimensionen benötigt, die aufgrund der großen Mengen an Gelmaterial und auf Grund der komplexeren technischen Ausrüstung ein teureres Verfahren bedingen. Für die großtechnische Produktion können Elutionen über beispielsweise Salzgradienten auch einen Engpass darstellen. Teilweise müssen auch harsche chemische Bedingungen, die die Funktionalität von Fibrinogen beeinträchtigen können, eingesetzt werden, um das gebundene Fibrinogen wieder zu eluieren. Zum Teil sind die beschriebenen Reinigungen auch mit hohen Verlusten an Fibrinogen verbunden oder aber nicht wirtschaftlich für die großtechnische Aufreinigungen von Fibrinogen einsetzbar z. B. aufgrund teurer oder nicht kommerziell erhältlicher Gelmaterialien. Gegenstand der Erfindung ist ein Verfahren nach Anspruch 1.

Die folgenden Ausführungsformen haben sich als vorteilhaft erwiesen:

Verfahren wie vorangehend beschrieben wobei als Farbstoffgel ein blaues Farbstoffgel verwendet wird.

Verfahren wie vorangehend beschrieben wobei als Farbstoffgel ein rotes oder grünes Farbstoffgel verwendet wird.

Verfahren wie vorangehend beschrieben wobei es sich bei dem Farbstoffgel um Blue Hyper D, Mimetic Blue Agarose, Mimetic Blue SA P6XL, Mimetic Blue 1 P6XL, Blue

Trisacryl Plus LS, Blue Uniflow, Blue Sepharose 6FF, Blue Sepharose CL 6B, Red Sepharose CL 6B, Fractogel TSK AF Green und/oder Matrex gel Green A handelt.

Verfahren wie vorangehend beschrieben wobei während des Verfahrensschrittes mit negativer Chromatographie und/oder negativer Adsorption ein pH-Wert zwischen 6 und 9 verwendet wird.

Verfahren wie vorangehend beschrieben wobei der Verfahrensschritt mit negativer Chromatographie und/oder negativer Adsorption bei einer Temperatur von 0-30°C durchgeführt wird.

Verfahren wie vorangehend beschrieben wobei die Ausbeute von Fibrinogen im Durchlauf der negativen Chromatographie oder im Überstand der negativen Adsorption ≥ 50%, bevorzugt ≥ 70% ist.

Verfahren wie vorangehend beschrieben wobei der Verfahrensschritt, der die negative Adsorption oder Chromatographie beinhaltet, in Anwesenheit von Substanzen, die die Bindung von Plasminogen an Fibrinogen schwächen, durchgeführt wird.

Verfahren wie vorangehend beschrieben wobei als Ausgangsmaterial eine aus Blut gewonnene Mischung, Milch von transgenen Tieren oder ein Fermentationsüberstand oder eine daraus hergstellte Fraktion verwendet wird.

Verfahren wie vorangehend beschrieben wobei als Ausgangsmaterial humanes Plasma, eine Plasmafraktion oder Kryopräzipitat verwendet wird.

Verfahren wie vorangehend beschrieben wobei ein oder mehrere Verfahrensschritte eine Aluminiumhydroxidbehandlung umfassen.
Verfahren wie vorangehend beschrieben wobei ein oder mehrere Verfahrensschritte enthalten sind, bei denen Fibrinogen präzipitiert wird.

Verfahren wie vorangehend beschrieben wobei ein oder mehrere Präzipitationen mit Glycin oder anderen Aminosäuren enthalten sind.

Verfahren wie vorangehend beschrieben wobei ein oder mehrere Verfahrensschritte enthalten sind, bei denen Plasminogen über Gelmaterial mit Lysin oder Lysin-Analoga als funktionelle Gruppe entfernt wird.

Verfahren wie vorangehend beschrieben wobei ein oder mehrere Verfahrensschritte zur Abreicherung und/oder Inaktivierung infektiöser Partikel enthalten sind.

Verfahren wie vorangehend beschrieben wobei es sich bei einem Verfahrensschritt um eine Pasteurisierung und/oder UV-Bestrahlung und/oder eine Nanofiltration handelt.

Verfahren wie vorangehend beschrieben wobei ein oder mehrere Ultrafiltrationen und/oder Dialysen enthalten sind.

Verfahren wie vorangehend beschrieben wobei Filtermaterialien mit einer Molekulargewichtsgrenze (cut off) von 100 bis 500 kDa verwendet werden.

Verfahren wie vorangehend beschrieben wobei ein oder mehrere Sterilfiltrationen enthalten sind.

Verfahren wie vorangehend beschrieben wobei folgende Verfahrensschritte kombiniert werden: Herstellung einer Plasmafraktion, Adsorption an Aluminiumhydroxid, Inaktivierung infektiöser Partikel wie beispielsweise Viren, Präzipitation, weitere Reinigungs- und/oder Inaktivierungsschritte, negative Chromatographie und/oder negative Adsorption, Ultrafiltration, Sterilfiltration.

Verfahren wie vorangehend beschrieben wobei es sich bei dem weiteren Reinigungsschritt um einen Verfahrensschritt zur Abtrennung von Plasminogen handelt.

Verfahren wie vorangehend beschrieben wobei die Reihenfolge und/oder die Anzahl der einzelnen Verfahrensschritte verändert ist.

Verfahren wie vorangehend beschrieben wobei einer der erfindungsgemäßen Verfahrensschritte verwendet wird und die abzutrennenden Plasmaproteine nach

Durchführung der negativen Chromatographie und/oder negativen Adsorption von dem Farbstoffgel eluiert werden.

Verfahren wie vorangehend beschrieben zur Isolierung von FXII, Plasminogen, t-PA und/oder F XI.

Weitere Ausführungsformen betreffen den Gegenstand der Ansprüche und weitere Merkmale und Vorteile der Erfindung gehen aus der Beschreibung der bevorzugten Ausführungsformen, den Beispielen und Figuren hervor.

Es folgt eine kurze Beschreibung der Figuren:

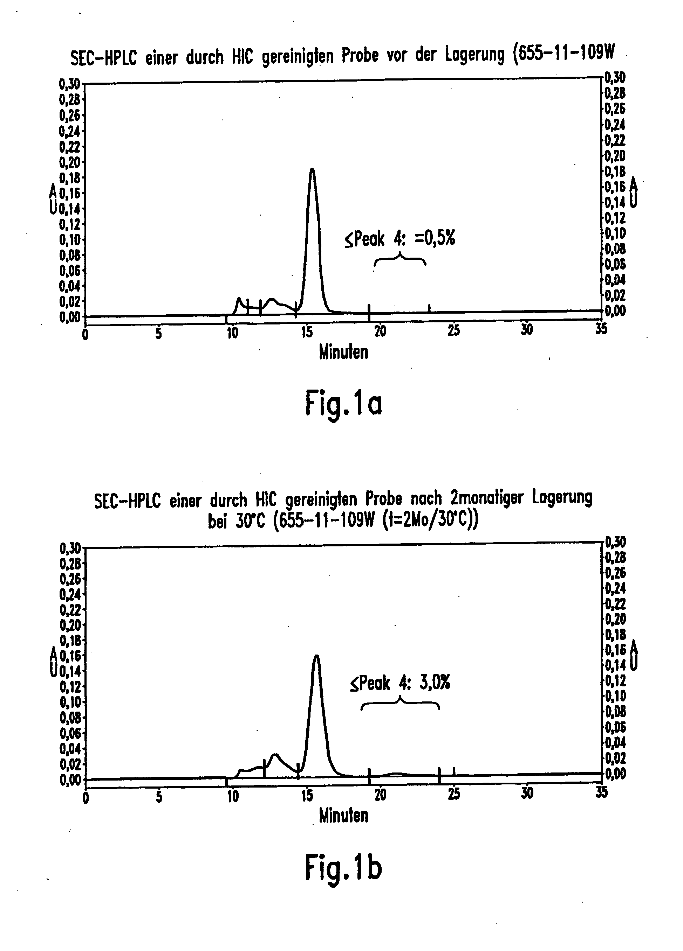

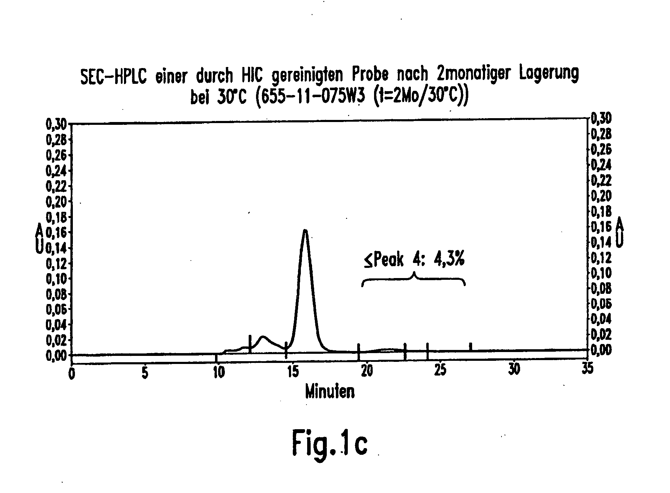

Figur 1 zeigt die Untersuchung fibrinogenhaltiger Lösungen auf Fibrinogen-Abbaufragmente mittels SEC-HPLC. Es wird gezeigt, dass durch Verwendung einer negativen hydrophoben Interaktionschromatographie der Anteil an Fibrinogen-Abbaufragmenten bei zweimonatiger Lagerung bei 30°C signifikant reduziert werden kann und die Stabilität von Fibrinogen in Lösung somit erhöht wird.
Figur 1a: Fibrinogenhaltige Lösung, die zusätzlich über eine hydrophobe Interaktionschromatographie im negativen Chromatographie-Modus aufgereinigt wurde, vor Beginn der Lagerung.
Figur 1b: Fibrinogenhaltige Lösung, die zusätzlich über eine hydrophobe Interaktionschromatographie im negativen Chromatographie-Modus aufgereinigt wurde, nach 2 monatiger Lagerung bei 30°C.
Figur 1c: Fibrinogenhaltige Ausgangslösung, ohne zusätzliche Reinigung über eine hydrophobe Interaktionschromatographie, nach 2 monatiger Lagerung bei 30°C.

Unter dem Begriff Fibrinogen ist vorzugsweise humanes Fibrinogen zu verstehen, das z.B. aus einer aus humanem Blut gewonnenen Mischung, die Fibrinogen enthält, aufgereinigt werden kann. Unter dem Begriff "aus Blut gewonnene Mischung" ist beispielsweise Gesamtblut, Blutplasma, Plasmafraktionen oder Plasmapräzipitate zu verstehen. Insbesondere bevorzugt ist Fibrinogen aus humanem Plasma, Cryopräzipitat oder Cohn Fraktion 1. Fibrinogen kann sowohl aus gepoolten Plasmaspenden als auch aus Einzelspenden isoliert sein. Humanes Fibrinogen kann auch aus der Milch transgener Tiere gewonnen werden, siehe beispielsweise US 5,639,940. Auch Fibrinogen, das durch rekombinante Expression aus Zellkultur gewonnen wird, siehe beispielsweise US 6,037,457, ist umfasst. So ist es möglich, Fibrinogen aus den entsprechenden Fermentationsüberständen oder daraus hergestellten Fraktionen zu isolieren. Umfaßt ist aber auch Fibrinogen isoliert aus einer aus tierischem Blut gewonnen Mischung, die Fibrinogen enthält, vorzugsweise von Tieren wie Säugetieren (z.B. Schwein, Pferd, Rind, Ziege, Schaf und Hund).

Unter kontaminierenden Proteinen im Sinne der Erfindung sind grundsätzlich alle Proteine zu verstehen, die im Plasma neben Fibrinogen vorkommen oder in der Milch transgener

Tiere oder im Zellkulturüberstand auftreten. Besonders bevorzugt handelt es sich um fibrinogenabbauende Proteine, die Fibrinogen proteolytisch abbauen können oder um Vorstufen der fibrinogenabbauenden Proteine (Proenzyme), die für einen proteolytischen Abbau von Fibrinogen zuvor aktiviert werden müssen, oder um Aktivatoren von fibrinogenabbauenden Proteasen. Bei einem proteolytischen Prozess entstehen Fibrinogen-Abbaufragmente, die kleiner als Fibrinogen sind und niedermolekular sein können. Dabei können die alpha und/oder beta und/oder gamma Ketten von Fibrinogen vom proteolytischen Abbau betroffen sein. Mögliche fibrinogenabbauende oder den Abbau unterstützende Proteine oder deren Vorstufen, die als mögliche Kontaminanten bei der Reinigung von Fibrinogen aus Plasma auftreten können, sind beispielsweise Plasmin, F Xla, Kallikrein, Faktor VII aktivierende Protease (FSAP), F Xlla, Plasminogenaktivatoren wie t-PA und u-PA, Thrombin, Metalloproteasen (MMP's) bzw. die entsprechenden Vorstufen wie z.B. Plasminogen, FXI, Präkallikrein, sc-FSAP, F XII, Einketten-Plasminogenaktivatoren wie sct-PA und scu-PA, Prothrombin und Pro-MMP's. Im folgenden wird nicht mehr unterschieden zwischen den aktivierten Formen und den jeweiligen Vorstufen, sondern die Bezeichnung der nicht aktivierten Proenzyme stellvertretend für beide Formen verwendet.

Das erfindungsgemäße Verfahren ermöglicht durch die/den erfindungsgemäße(n) Vefahrensschritt(e) vorzugsweise eine Abreicherung von fibrinogenabbauenden Proteasen oder deren Proenzymen und Aktivatoren. Diese können z.B. Plasminogen, FXI, Präkallikrein, F XII, Pro-MMP's und/oder Plasminogenaktivatoren sct-PA und scu-PA sein. Der Abreicherungsfaktor (AF) liegt oberhalb von 1, vorzugsweise oberhalb von 2. Dabei kann der Anteil von F XII insbesonders bevorzugt auf ≤ 20 ng pro OD₂₈₀₋₃₂₀ minimiert werden. Der Anteil von F XI kann auf ≤ 1 ng pro OD₂₈₀₋₃₂₀, insbesonders bevorzugt auf ≤ 0,2 ng pro OD₂₈₀₋₃₂₀ minimiert werden. Der Anteil von Plasminogen kann besonders bevorzugt bis auf Werte von ≤ 5 ng pro OD₂₈₀₋₃₂₀ verringert werden. Besonders bevorzugt wird durch den erfindungsgemäßen Verfahrensschritt die Stabilität von Fibrinogen in Lösung und insbesondere die proteolytische Stabilität der Fibrinogen-Ketten erhöht. Die durch die Proteolyse von Fibrinogen durch fibrinogenabbauende Proteine entstehenden Abbaufragmente lassen sich durch unterschiedliche Methoden nachweisen. Beispielhaft genannt seinen hier die Auftrennung in SDS-Polyacrylamidgelen unter reduzierenden oder nicht reduzierenden Bedingungen, HPLC-Methoden wie beispielsweise die SEC (Size Exclusion Chromatography)-HPLC oder immunologische Methoden. Die Aktivität von verbleibendem Fibrinogen kann weiterhin nach gängigen Methoden wie bei Clauss (Acta-Haematol. 17 (1957) 237-246) beschrieben, bestimmt werden. Der Nachweis der Abreicherung von fibrinogenabbauenden Proteinen kann beispielsweise spezifisch mit Hilfe von Antikörpern erfolgen, wie z.B. im bekannten ELISA (enzyme-linked immunosorbent assay) oder RIA (radioimmunoassay) Verfahren. Bei den Antikörpern handelt es sich z.B. um Antikörper, die u.a. gegen die genannten bzw. weitere bekannte, fibrinogenabbauende Proteasen gerichtet sind. Der Nachweis der Abreicherung kann aber unspezifisch auch dadurch erfolgen, dass die Fibrinogenlösung im flüssigen Zustand gelagert wird und der Grad einer eventuellen Abbaureaktion nach Lagerung im Vergleich zu einer ebenfalls gelagerten, nicht-abgereicherten Kontrolle bestimmt wird.

Unter Chromatographie im Sinne dieser Erfindung ist eine Trennmethode zu verstehen, bei der eine fibrinogenhaltige Lösung mit Hilfe eines Flüssigkeitsstromes über eine stationäre Phase geleitet wird, und Bestandteile der Mischung sich auftrennen. Bei der stationären Phase handelt es sich vorzugsweise um das Füllmaterial einer chromatographischen Säule. Das Füllmaterial, nachfolgend auch Gelmaterial genannt, besteht aus einem festen Trägermaterial aus vorzugsweise etwa gleich großen, porösen oder nicht porösen Partikeln, an dem sich kovalent gebundene funktionelle Gruppen, die den Trennmodus festlegen, befinden. Bei dem Trägermaterial kann es sich beispielsweise um Biopolymere wie Agarose, Cellulose und Dextran (vorzugsweise Sepharose und Sephadex), oder synthetische Polymere wie z. B. Methacrylate, Polyvinylbenzol, Polystyrol und Polyacrylamid oder anorganische Polymere, wie z. B. Kieselsäure oder poröse Glaskügelchen, handeln. Die Lösung, in der das zu reinigende Fibrinogen mit kontaminierenden Proteinen gelöst ist, sowie mögliche Waschpuffer und Äquilibrierungspuffer sind als mobile Phasen zu betrachten. Unter negativer Chromatographie im Sinne dieser Erfindung ist zu verstehen, dass die stationäre und die mobile Phase so zu wählen sind, dass einerseits Fibrinogen möglichst schwache oder keine Wechselwirkung mit der stationären Phase eingeht, während andererseits ein oder mehrere kontaminierende Proteine stärkere Wechselwirkungen mit der stationären Phase eingehen als Fibrinogen. Das Fibrinogen passiert somit vorwiegend die Säule und befindet sich vorwiegend im Durchlauf (> 50%), während ein oder mehrere kontaminierende Proteine vorwiegend an der stationären Phase gebunden vorliegen. Die Säule wird vor der Beladung mit der fibrinogenhaltigen Lösung entsprechend den Erfordernissen des Gelmaterials vorbehandelt und äquilibriert. Der Äquilibrierungspuffer entspricht vorzugsweise der Lösung, in der Fibrinogen und die kontaminierenden Proteine gelöst sind. Um Verluste an Fibrinogen zu minimieren, kann in der mobilen Phase der Säule noch verbliebenes Fibrinogen herausgewaschen werden, wobei das Volumen des Waschpuffers vorzugsweise etwa dem des Säulenvolumens entspricht. Der Waschpuffer entspricht vorzugsweise der Lösung, in der das Fibrinogen und die kontaminierenden Proteine gelöst sind. Der Waschpuffer kann gegebenenfalls mit dem Durchlauf vereinigt werden. Die an der stationären Phase gebundenen, kontaminierenden Proteine können mit entsprechenden Lösungen eluiert werden und die stationäre Phase durch Regeneration aus Kostenersparnisgründen gegebenenfalls mehrfach für einen negativen chromatographischen Reinigungsschritt verwendet werden. Ist eine Regeneration nicht möglich oder nicht wirtschaftlich, wird das gebrauchte Gelmaterial verworfen. Die von der stationären Phase eluierten Proteine können bei Bedarf aber auch als Ausgangsmaterial für die Isolierung dieser Proteinkomponenten genutzt werden. Durch gegebenenfalls weitere Reinigungs- bzw. Verfahrensschritte können Proteinkonzentrate gewonnen werden. Auf diese Art und Weise können z. B. Plasminogen-, F XII-, F XI- u.a. Konzentrate hergestellt werden.

Bei der negativen Chromatographie im Sinne dieser Erfindung kann grundsätzlich für die stationäre Phase jedes Material verwendet werden, das unter den gewählten Bedingungen, insbesondere der Wahl der mobilen Phase, in der Lage ist, mit ein oder mehreren kontaminierenden Proteinen stärkere Wechselwirkungen einzugehen als mit dem Fibrinogen. Umfasst sind für den erfindungsgemäßen Verfahrensschritt Gelmaterialien (stationäre Phasen), die zu den Gruppen der Kationenaustauscher, hydrophoben Gele oder Farbstoffgele zählen. Es gibt ein breites Angebot kommerziell erhältlicher Säulenmaterialien oder fertig gepackter Säulen, z.B. bei Amersham, Bio-Rad, Biosepra, Merck, Perseptive Biosystems, Pharmacia, Prometic, Toso Haas, die als umfasst anzusehen sind. Einige wurden beispielhaft unter den aufgeführten Beispielen getestet. Die mobilen Phasen sind den jeweiligen Gelmaterialien anzupassen. Erfindungsgemäß sind die pH-Werte im Bereich zwischen 5.5 und 9. Entsprechend sind Puffersysteme zu wählen, die diesen Bereich gut abpuffern. Beispiele wären Citrat, Phosphat und Tris Puffer.

Der Verfahrensschritt der negativen Chromatographie wird vorzugsweise bei einer Temperatur zwischen 2 und 30 °C durchgeführt.

Kationenaustauscher sind Gelmaterialien der Ionenaustauschchromatographie, bei der Proteine mit Salzionen der mobilen Phase um die geladenen Positionen auf einer stationären Phase, der lonenaustausch-Matrix, konkurrieren. Kationenaustauscher enthalten negativ geladene Gruppen und können deshalb mit positiv geladenen Gruppen von Proteinen interagieren. Die Interaktion ist abhängig von der Stärke der positiven Ladung und der Ladungsdichte auf der Oberfläche des Kationenaustauschers sowie des Proteins. Proteine tragen positive oder negative Ladungen aufgrund basischer und saurer Seitengruppen von bestimmten Aminosäuren, wobei der Gesamtladungszustand vom pH-Wert der Lösung abhängt. Neben der Aminosäurezusammensetzung tragen aber auch posttranslationale Modifikationen (wie beispielsweise Phosphorylierungen) zum isoelektrischen Punkt (pl) eines Proteins bei, bei dem sich positive und negative Ladungen neutralisieren. Fibrinogen gehört mit einem pl von ca. 5.1 - 5.5 zu den eher sauren Proteinen. Bevorzugte Kationenaustauscher enthalten als Austauschfunktion (funktionelle Gruppe, Liganden) Sulfomethylgruppen (S-Typen), Sulfopropylgruppen (SP-Typen) oder Carboxymethylgruppen (CM-Typen). Zu dem Bereich der Kationenaustauscher zählen im Sinne dieser Erfindung aber auch solche mit anderen, geeigneten negativ geladenen funktionellen Gruppen wie beispielsweise Heparin. Kationenaustauscher sind beschrieben und kommerziell erhältlich über Firmen wie beispielsweise Amersham, Bio-Rad, Biosepra, Merck, Perseptive Biosystems, Pharmacia, Prometic oder Toso Haas. Bevorzugte Kationenaustauscher sind in situ sanitisierbare Gele wie z.B. Fractogel EMD SO₃⁻ 650 (Merck), Macro-Prep 50 S (Bio-Rad), CM-Sepharose CL-6B (Pharmacia), Fractogel TSK CM 650 (Merck), Fractogel EMD CM, SP Sepharose, Heparin Fractogel (Merck) oder Heparin-Sepharose CL 6B (Pharmacia).

Die mobile Phase beim Kationenaustauscher sollte vorzugsweise so gewählt werden, dass das Fibrinogen bei dem gewählten pH-Wert eine negative Gesamtladung besitzt und/oder bei der gewählten lonenstärke keine oder nur geringe Wechselwirkungen mit den negativ geladenen Gruppen der stationären Phase auftreten, während zwischen der stationären Phase und ein oder mehreren kontaminierenden Proteinen noch Wechselwirkungen auftreten. Der pH-Wert sollte oberhalb von pH 5.5 liegen und insbesondere bevorzugt oberhalb von 6,0 und bei bis zu ca. 9 liegen. Als Salze können dem Fachmann für Kationenaustauscher bekannte Salze verwendet werden, insbesondere bevorzugt ist NaCl. Die Salzkonzentration liegt bevorzugt innerhalb 0 und 400 mM, insbesonders bevorzugt innerhalb von 0 und 100 mM.

Eine hydrophobe Chromatographie ist **dadurch gekennzeichnet, dass** die unpolaren Oberflächenregionen eines Proteins bei meist höheren Salzkonzentrationen mit hydrophoben funktionellen Gruppen (Liganden) der stationären Phase in Wechselwirkung treten. Das hydrophobe Gel als stationäre Phase ist vorzugsweise ein synthetisches Polymer, Silikat oder ein Biopolymer wie z.B. Sepharose, deren Oberflächen durch hydrophobe Liganden als funktionelle Gruppe modifiziert sind. Bei den hydrophoben Liganden handelt es sich vorzugsweise um Alkyl-Gruppen mit 1 bis mehr als 24 Kohlenstoffatomen (C), die linear oder verzweigt sein können, oder um aromatische Liganden. Möglich sind beispielsweise C1 (Methyl), C3 (Propyl), C4 (Butyl), C5 (Pentyl), C6 (Hexyl) und C8 (Octyl) Gruppen, insbesonders bevorzugt sind Propyl-, Butyl-, Pentyl-, Hexyl- und Octyl-Gruppen. Die Alkylgruppen können derivatisiert sein, wie beispielsweise Thiopropyl-Gruppen. Vorzugsweise können auch Phenyl-Gruppen oder aromatische Verbindungen, die ein Phenyl-Derivat enthalten, wie z. B. Phenylalanin-Gruppen, verwendet werden. Phenyl-Gruppen können beispielsweise auch mit Alkylketten verbunden sein. Verwendet werden können beispielsweise auch Pyridyl-Gruppen oder deren Derivate. Hydrophobe Materialien sind dem Fachmann weitreichend bekannt und kommerziell erhältlich beispielsweise bei den Firmen Amersham, Bio-Rad, Biosepra, Merck, Perseptive Biosystems, Pharmacia, Prometic und Toso Haas. Als stationäre Phase insbesonders bevorzugt sind z.B. Macro Prep Methyl HIC Support (Bio-Rad), Fractogel EMD Propyl 650 (Merck), Fractogel EMD Butyl 650 (Merck), Fractogel TSK Butyl 650 (Merck), Macro Prep t Butyl HIC Support (Bio-Rad), Butyl Cellufine (Amicon), Butyl Sepharose 4 Fast Flow (Pharmacia), Butyl S-Sepharose 6 Fast Flow (Prototyp, Pharmacia), HIC-Fractogel Pentyl (Merck), Hexyl S-Sepharose 6 Fast Flow (Prototyp, Pharmacia), Octyl Sepharose CL 4B (Pharmacia), Fractogel HW 65 Propyltentakel (Merck), Fractogel HW 65 Butyltentakel (Merck), Fractogel TA 650 (Merck), Phenyl Sepharose High Performance (Pharmacia), Phenyl Sepharose Fast Flow (Pharmacia), Phenylalanin Sepharose (Pharmacia), Thiopropyl Sepharose 6B (Pharmacia) oder Pyridyl S-Sepharose 6 Fast Flow (Pharmacia).

Die hydrophobe Wechselwirkung der Proteine zur stationären Phase wird nicht nur durch die funktionellen Gruppen beeinflusst (z. B. erhöht steigende Kettenlänge von Alkylgruppen den hydrophoben Charakter), sondern auch sehr stark durch lonenstärke und pH-Wert der Lösung, die die mobile Phase bildet. In Abhängigkeit von der verwendeten stationären Phase ist die mobile Phase so zu wählen, dass ein oder mehrere kontaminierende Proteine hydrophobe Wechselwirkungen mit der stationären Phase eingehen, während Fibrinogen bevorzugt zu mehr als 50% die Säule passiert. Dem Fachmann sind mögliche mobile Phasen und Anpassungen an die jeweilige stationäre Phase bekannt. Der pH-Wert sollte bevorzugt im Bereich oberhalb von ca. 5 und bis zu ca. 9 liegen. Es können für die hydrophobe Interaktionschromatographie bekannte Salze verwendet werden, insbesondere bevorzugt sind z.B. NaCl, Na₂SO₄ und (NH₄)₂SO₄. Die Salzkonzentration liegt, in Abhängigkeit von der stationären Phase, bevorzugt im Bereich von 0,01 bis 2 M.

Eine Affinitätschromatographie ist **dadurch gekennzeichnet, dass** es zur spezifischen, reversiblen Adsorption eines Proteins (oder einer Proteingruppe) an einen individuellen Liganden, der an die Trägermatrix gebunden ist, kommt. Eine geeignete Trägermatrix ist z. B. Sepharose wie Sepharose 4B oder Sepharose CL, deren OH-Gruppen zur kovalenten Bindung des Liganden herangezogen werden können. Andere geignete Träger sind bekannte Polymere wie z. B. Fractogel, Sephacryl, Cellufine o.ä.. Mögliche monospezifische Liganden sind z. B. Antikörper, die gegen fibrinogenabbauende Proteine wie z. B. Plasminogen, F XI, Präkallikrein oder andere gerichtet sind. Affinitätssäulen mit Antikörpern sind im großtechnischen Maßstab aber teuer und wirtschaftlich nur bedingt effektiv einsetzbar. So sind im Rahmen dieser Erfindung Farbstoffe als gruppenspezifische Liganden umfasst, die ein oder mehrere kontaminierende Proteine, insbesondere fibrinogenabbauende Proteine binden. Insbesonders bevorzugt sind blaue, rote und grüne Farbstoffgele. Entsprechende Materialien sind z. B. Blue Sepharose 6FF (Pharmacia), Blue Sepharose CL 6B (Pharmacia), Blue Trisacryl Plus LS (Biosepra/Ciphergen), Blue Hyper D (Biosepra/Ciphergen), Mimetic Blue Agarose (Prometic), Mimetic Blue SA P6XL (Prometic), Mimetic Blue 1 P6XL (Prometic), Red Sepharose CL 6B (Pharmacia), Fractogel TSK AF Green (Merck) und/oder Matrex gel Green A (Amicon). Weitere Farbstoffgele sind dem Fachmann weitreichend bekannt und kommerziell bei den einschlägigen Herstellern erhältlich oder herstellbar.

Der pH-Wert der mobilen Phase liegt im Bereich von 5.5 bis 9.

Unter negativer Adsorption im Sinne dieser Erfindung ist zu verstehen, dass eine fibrinogenhaltige Lösung in einem geeigneten Behälter mit einem Adsorptionsmaterial gemischt wird, das unter den gewählten Bedingungen Fibrinogen nicht oder nur zu geringen Mengen adsorbiert, ein oder mehrere kontaminierende Proteine hingegen adsorbiert werden. Fibrinogen verbleibt damit überwiegend in der Lösung. Dieses Verfahren ist auch als diskontinuierliches oder Batch-Verfahren bekannt. Die Bedingungen der Adsorption sind so zu wählen, dass die kontaminierenden Proteine genügend Zeit und Möglichkeit haben, an das Adsorptionsmaterial zu binden. Beispielsweise kann eine Mischung z. B. durch vorsichtiges Rühren bei geeigneten Temperaturen erfolgen. Geeignete Temperaturen liegen beispielsweise zwischen 2 und 30°C. Als Adsorptionsmaterial kann grundsätzlich jedes Material eingesetzt werden, das unter den gewählten Bedingungen in der Lage ist, ein oder mehrere kontaminierende Proteine zu binden und Fibrinogen nicht oder nur zu geringen Anteilen zu binden. Umfasste Materialien sind Gelmaterialien, die bereits unter der Chromatographie aufgeführt wurden und somit zu den Gruppen der Kationenaustauscher, Farbstoffgele und hydrophoben Gele zählen. Bevorzugte Lösungen entsprechen bevorzugten mobilen Phasen bei der Chromatographie. Das Adsorptionsmaterial mit den gebundenen, kontaminierenden Proteinen kann durch dem Fachmann bekannte Methoden von der fibrinogenhaltigen Lösung abgetrennt werden. Vorzugsweise genannt seien hier die Methode der Filtration, der Zentrifugation und/oder Sedimentation. Das Adsorptionsmaterial kann bevorzugt durch Elution der Kontaminanten und Umpuffern regeneriert werden, so dass es mehrfach verwendet werden kann. Die von dem Adsorptionsmaterial eluierten Proteine können bei Bedarf auch als Ausgangsbasis für die Isolierung dieser Proteinkomponenten genutzt werden. Durch gegebenenfalls weitere Reinigungs- bzw. Verfahrensschritte können so Proteinkonzentrate gewonnen werden. Auf diese Art und Weise können z.B. Plasminogen-, F XII-, F XI- u.a. Konzentrate hergestellt werden.

Die negative Adsorption im Batch-Format ist eine Technik, die zusätzlich oder anstelle der negativen Chromatographie verwendet werden kann.

Die erfindungsgemäßen Verfahrensschritte ermöglichen in der Regel eine Schrittausbeute von ≥ 50% Fibrinogen im Durchlauf bzw. im Überstand, vorzugsweise von ≥ 70 %.

In einer weiteren Ausführungsform werden während des erfindungsgemäßen Verfahrens und insbesondere während der erfindungsgemäßen Verfahrenschritte Substanzen zugesetzt, die die Bindung von Plasminogen an Fibrinogen schwächen. Es ist bekannt, dass Fibrinogen dazu tendiert, andere Proteine wie beispielsweise Plasminogen zu binden. Dies führt immer wieder zu teilweiser Coaufreinigung von Kontaminanten wie Plasminogen. Durch die Bindung schwächende Substanzen lassen sich Coaufreinigungen minimieren. Zu diesen Substanzen zählen vorzugsweise ω-Aminosäuren wie die ε-Aminocapronsäure, weiterhin Tranexamsäure, PAMBA (p-aminomethylbenzoic acid), Lysin und weitere Lysinanaloga.

Es ist bekannt, dass die Anreicherung und Isolierung von Fibrinogen im Regelfall mehrere Verfahrensschritte benötigt und es zahlreiche Kombinationsmöglichkeiten dieser einzelnen Reinigungschritte gibt. Das erfindungsgemäße Verfahren kann neben ein oder mehreren erfindungsgemäßen Verfahrensschritt(en) also zusätzliche Verfahrensschritte enthalten, die grundsätzlich dem Stand der Technik für die Reinigung von Fibrinogen zu entnehmen sind und hiermit umfasst sind. Der Stand der Technik zur Aufreinigung von Fibrinogen wurde bereits im Wesentlichen dargelegt und die entsprechend zitierten Patentschriften und Publikationen sind hiermit umfasst. Bevorzugt werden Verfahrensschritte, die keine Chromatographie oder Adsorption beinhalten, bei der Fibrinogen zum großen Teil bindet und damit eine Elution mit all ihren Nachteilen erforderlich würde.

In einer bevorzugten Ausführungsform enthält das erfindungsgemäße Verfahren ein oder mehrere Verfahrensschritte, in denen Aluminiumhydroxid (Al(OH)₃) der zu reinigenden fibrinogenhaltigen Lösung zugesetzt wird. Das Al(OH)₃ adsorbiert primär die Faktoren des Prothrombinkomplexes und kann beispielsweise durch Zentrifugation und/oder Filtration entfernt werden.

In einer bevorzugten Ausführungsform enthält das Verfahren ein oder mehrere Verfahrensschritte, in denen das Fibrinogen präzipitiert wird. Insbesondere bevorzugt ist die Zugabe von Glycin oder anderen Aminosäuren zur Fällung. Bei genügend hoher Glycinkonzentration führt dies zu einer direkten Präzipitation von Fibrinogen (einstufige Glycinpräzipitation). Möglich ist aber alternativ oder zusätzlich eine zweistufige Glycinpräzipitation, bei der die Glycinkonzentration in einem ersten Schritt so gewählt wird, dass Fibrinogen im wesentlichen im Überstand verbleibt und präzipitierte Proteine durch z.B. Zentrifugation abgetrennt werden und erst in einem zweiten Schritt die Hauptmenge des Fibrinogens durch eine Erhöhung der Glycinkonzentration, wie auch in den Beispielen gezeigt, präzipitiert wird. Wie oben erwähnt können anstelle der Aminosäure Glycin auch andere Aminosäuren verwendet werden. Beispielhaft seien hier Alanin, Glutamin oder Glutaminsäure genannt. Aber auch andere bekannte Fällungsagentien (wie z.B. Natriumchlorid, Ammoniumsulfat oder Polyethylenglykol) können hierbei verwendet werden.

In einer weiteren bevorzugten Ausführungsform enthält das Verfahren ein oder mehrere Verfahrensschritte, bei denen Plasminogen über ein Gelmaterial mit Lysin-Liganden oder analogen Liganden abgereichert wird. Beispiele wären hier die Lysin-Sepharose, Lys-Hyper D, Lys-Fractogel, Aminohexyl-Sepharose o.a.. Dies führt zu einer vorteilhaften Abreicherung von Plasminogen, die sich zusätzlich vorteilhaft auf die Stabilität von Fibrinogen bei Flüssiglagerung auswirken kann. Darüber hinaus ist eine Abreicherung von Plasminogen auch vorteilhaft, wenn das Fibrinogenkonzentrat als Komponente eines Fibrinklebers eingesetzt werden soll.

In einer weiteren bevorzugten Ausführungsform beinhaltet das Verfahren ein oder mehrere Verfahrensschritte, in dem potentiell vorhandene infektiöse Partikel wie z.B. Viren inaktiviert oder möglichst weitgehend abgereichert werden. Wird das Fibrinogen aus humanem Plasma isoliert, ist dies ein besonders bevorzugter Bestandteil des erfindungsgemäßen Verfahrens. Inaktivierung oder Abreicherung von infektiösen Partikeln kann nach dem Fachmann bekannten Techniken, die hiermit umfasst sind, erfolgen. Dabei handelt es sich um Verfahren wie beispielsweise Pasteurisierung, Erhitzung im trockenen Zustand, Nanofiltration, chemische Zusätze (beispielsweise Detergenzien), Bestrahlungen oder Kombinationen davon.

In einer weiteren bevorzugten Ausführungsform enthält das Verfahren ein oder mehrere Verfahrensschritte, die eine Ultrafiltration und/oder Dialyse beinhalten. Diese Methoden werden vorteilhaft eingesetzt, um die fibrinogenhaltige Lösung umzupuffern, d.h. Lösungsbestandteile zu verändern bzw. die Proteinlösung anzukonzentrieren. Dies ist insbesondere bevorzugt zur Vorbereitung auf einen Reinigungsschritt oder am Ende des erfindungsgemäßen Verfahrens, um Formulierungsbestandteile zu wählen, die für die Lagerung und Verwendung als pharmazeutische Zubereitung geeignet sind.

Die Ultrafiltration bietet auch die Möglichkeit, zusätzlich kontaminierende Proteine abzureichern, indem Filter gewählt werden, die kontaminierende Proteine passieren lassen, Fibrinogen aber zurückhalten, wie beispielsweise auch in der WO 93/17776 beschrieben. Im Zuge dieser Erfindung konnte gezeigt werden, dass bei Auswahl geeigneter Filter mit einer Molekulargewichtsgrenze (cut off) von z.B. 300 kDa auch eine Abreicherung von fibrinogenabbauenden Proteinen möglich ist. In einer besonders bevorzugten Ausführungsform werden für die Ultrafiltration also Filter mit einer Molekulargewichtsgrenze (cut off) von 50 bis 500 kDa eingesetzt.

In einer weiteren bevorzugten Ausführungsform enthält das Verfahren eine Sterilfiltration. Dies ist insbesondere sinnvoll zum Abschluss des Verfahrens zur Herstellung einer pharmazeutischen Zubereitung.

In einer bevorzugten Ausführungsform werden mehrere der oben angeführten Verfahrensschritte mit ein oder mehreren erfindungsgemäßen Verfahrensschritten zu einem erfindungsgemäßen Verfahren kombiniert. Die Reihenfolge einzelner Verfahrensschritte kann dabei variiert werden. Auch können einzelne Verfahrensschritte, wie z.B. die Präzipitation mehrfach zur Anwendung kommen. In einer insbesonders bevorzugten Ausführungsform enthält eine Kombination neben ein oder mehreren erfindungsgemäßen Verfahrensschritten mindestens die Schritte der Al(OH)₃ Adsorption, ein- und/oder zweistufige Glycinpräzipitationen, sowie die Pasteurisierung. Vorteilhaft werden diese Verfahrensschritte mit einer Affinitätssäule mit Lysin-Liganden kombiniert. Nach Abschluss der Reinigung werden die Lösungskomponenten durch eine Lösung ersetzt, die für die Lagerung geeignet ist. Mögliche Methoden wie beispielsweise Dialyse, Ultrafiltration, usw. sind dem Fachmann bekannt und hiermit umfasst. Eine Sterilfiltration kann an die Reinigung angeschlossen werden. Das erfindungsgemässe Reinigungsverfahren besteht in einer besonders bevorzugten Ausführungsform aus den folgenden Verfahrensschritten:
- Herstellung einer Plasmafraktion
- Adsorption an Aluminiumhydroxid
- Inaktivierung infektiöser Partikel wie beispielsweise Viren
- Präzipitation
- Weitere Reinigungs- und/oder Inaktivierungsschritte
- negative Chromatographie(n) und/oder negative Adsorption(en)
- Ultrafiltration / Diafiltation
- Sterilfiltration

In einer insbesonders bevorzugten Ausführungsform zählt zu den weiteren Reinigungsschritten eine Affinitätssäule mit Lysin-Liganden, wie beispielsweise Lys-Sepharose. Sowohl die Anzahl als auch die Reihenfolge einzelner Verfahrensschritte kann variiert werden. So könnte z.B. ein oder mehrere negative Adsorptionen oder Chromatographieschritte direkt im Anschluss an die Al(OH)₃ Adsorption erfolgen. Dies hätte den Vorteil, dass bereits zu einem frühen Zeitpunkt mögliche fibrinogenabbauende Proteine abgereichert werden, die somit im weiteren Reinigungsverlauf die Stabilität des Fibrinogens nicht mehr negativ beeinflussen können.

Bei Anwendung der besonders bevorzugten Ausführungsformen kann der FibrinogenAbbau bei Lagerung von Fibrinogen in flüssiger Form für 1 Monat bei 30°C in Abwesenheit zugesetzter Fibrinolyseinhibitoren signifikant reduziert werden, d.h. in dieser Lagerzeit werden signifikant weniger niedermolekulare Abbaufragmente erzeugt. Mit einer Analyse mittels SEC-HPLC lässt sich zeigen, dass die Zunahme des Anteils niedermolekularer Abbaufragmente im Vergleich zu den restlichen Peaks bei Anwendung der insbesonders bevorzugten Ausführungsformen, die zusätzlich eine Affinitätssäule mit Lysin-Liganden einsetzen, bei unter 2,5%, im besonderen bei unter 1,5%-2% liegt.

Das nach dem erfindungsgemäßen Verfahren gewonnene Fibrinogen bzw. eine entsprechende pharmazeutische Zubereitung enthält vorzugsweise nur noch ≤ 0,2 ng pro OD₂₈₀₋₃₂₀ an F XI und/oder ≤ 20 ng pro OD₂₈₀₋₃₂₀ an F XII und/oder ≤ 5 ng pro OD₂₈₀₋₃₂₀ an Plasminogen und/oder ≤ 0,02 ng, bevorzugt ≤ 0,01 ng pro OD₂₈₀₋₃₂₀ an t-PA.

Die während des erfindungsgemäßen Verfahrensschrittes zur Aufreinigung von Fibrinogen mittels Chromatographie oder Adsorption an Farbstoffsäulen gebundenen kontaminierenden Proteine können als Ausgangsbasis für die Isolierung dieser Protein-Komponenten dienen. Durch Elution der Proteine von dem entsprechenden Gelmaterial und gegebenenfalls weiteren Reinigungs- bzw. Verfahrensschritten können entsprechende Proteinkonzentrate gewonnen werden. Auf diese Art und Weise können z. B. Plasminogen-, t-PA-, F XII- oder F XI-Konzentrate hergestellt werden. Umfasst von dieser Anmeldung ist demnach ein Verfahren zur Isolierung ein oder mehrerer Plasmaproteine.

Weiterhin soll die Erfindung durch die folgenden Beispiele veranschaulicht werden, die jedoch in keiner Weise einschränkende Wirkung haben sollen.

### Beispiel 1:

In diesem Beispiel wird gezeigt, dass Kationenaustauschermaterialien sowie hydrophobe Gele und Farbstoffgele in der Lage sind, eine fibrinogenhaltige Lösung mittels negativer Chromatographie so aufzureinigen, dass die Stabilität des Fibrinogens gegenüber dem Ausgangsmaterial erhöht ist.

Für die Gewinnung von Fibrinogen-Ausgangsmaterial wurde wie in EP 0 103 196 beschrieben ein pasteurisiertes Fibrinogen Konzentrat hergestellt und durch Glycin-Zugabe fraktioniert gefällt.

Das fibrinogenreiche Präzipitat wurde zunächst in einem geeigneten wässrigen Lösungmittel (50 mM NaCl; 20 mM tri-Natriumcitrat-dihydrat, 0,05% NaN₃ pH 7,3) gelöst und diente als Ausgangsmaterial für die weiteren Reinigungsschritte unter Verwendung der negativen Chromatographie.

Die für diese Reinigung verwendeten Chromatographie-Säulen (∅ 0.7 cm) wurden jede mit 1,0 ml des jeweiligen Gelmaterials gefüllt. Die Säulen wurden in Abhängigkeit von dem jeweiligen Gelmaterial in einem der Puffer 1-3 äquilibriert.
Puffer 1: 50 mM NaCl, 50 mM Natriumphosphat pH 7,4 für Kationenaustauscher und Vergleichssäule
Puffer 2: 1000 mM NaCl, 50 mM Natriumphosphat pH 6,5 für hydrophobe Gele
Puffer 3: 50 mM Natriumphosphat pH 7,4 für Farbstoffgele

Je 30-40 ml fibrinogenhaltige Lösung wurden gegen die entsprechenden Puffer (1 - 3) dialysiert (siehe Tabelle 1) und mit den entsprechenden Puffern auf eine OD₂₈₀₋₃₂₀ von 10 verdünnt. 15 ml dieser Fibrinogen-Lösung wurden pro Säule aufgetragen und die Säulen mit je 1,0 ml des jeweiligen Puffers (1 - 3) gewaschen. Der Säulendurchlauf und die Waschlösung wurden vereinigt. Zunächst wurde die optische Dichte am Photometer bei 280 und 320 nm (OD₂₈₀₋₃₂₀) gemessen, um im Vergleich zum Ausgangsmaterial die Ausbeute (in %) zu bestimmen. Ein Aliquot dieses Materials wurde zur Bestimmung des 0-Wertes des anschließenden Lagerversuches für die Analyse zurückgehalten. Die mit den jeweiligen Waschlösungen vereinigten Durchläufe der Säulen und das Ausgangsmaterial wurden gegen einen Puffer enthaltend 100 mM NaCl, 20 mM Na₃-Citrat, 5% L-Arg pH 7,2 dialysiert und jeweils 1 ml davon mit Natrium-Azid (0,05% w/v Endkonzentration) versetzt. Die Lagerung dieser unterschiedlich aufgereinigten Fibrinogenzubereitungen sowie des Ausgangsmaterials als Kontrolle erfolgte bei +30°C für Lagerzeiten bis zu 3 Monaten. Die Stabilität von Fibrinogen wurde nach den entsprechenden Lagerzeiträumen jeweils mittels SEC-HPLC bestimmt. Dabei handelt es sich um eine Größenausschluss-Chromatography (SEC), die die vorliegenden Proteine und Spaltprodukte entsprechend ihres Molekulargewichtes auftrennt. Niedermolekulare Fragmente, die aufgrund eines proteolytischen Abbaus von Fibrinogen entstanden sind, finden sich als neuer Peak (Fragment Peak 4) sowie ggfs. weiteren neuen Peaks mit erhöhter Retentionszeit. Die Fläche der Fragment Peaks (≤ Peak 4) wurde bestimmt und als Prozentanteil aller Peaks berechnet Der Wert wurde um den geringen Anteil vor Beginn der Lagerung (Nullwert) bereinigt und die hieraus ermittelten Werte sind als Ergebnis in Tabelle 1 gezeigt. Sie geben den Anstieg an Abbaufragmenten während der Lagerung unter akzelerierten Bedingungen wieder.

Für die Durchführung der Analytik wurde ein entsprechendes HPLC-System mit einer SEC-Säule (TSK gel G 4000 SWXL, 7,5 x 300 mm von TOSO HAAS) verwendet. Die Auftrennung der Proteine und Proteinfragmente nach entsprechenden Lagerzeiten erfolgte bei einer Flussrate von 0,5 ml/min in einem geeigneten Laufpuffer bei 20-25 °C. Die Detektion der Protein- bzw. Proteinfragment-Peaks erfolgte bei 280 nm.

Die Figur 1 zeigt beispielhaft typische Trennverläufe von Fibrinogen mittels SEC HPLC. Sie zeigt die Auftrennung einer fibrinogenhaltigen Lösung, die mit Hilfe einer Lysin-Sepharose Chromatographie und einer negativen HIC gereinigt wurde vor Beginn der Lagerung (t=0, Nullwert, Figur 1a) und nach 2 Monaten Lagerung bei 30°C (t= 2 Monate, Figur 1b).

Figur 1c hingegen zeigt die Auftrennung einer fibrinogenhaltigen Lösung ohne zusätzliche Reinigung über negative HIC nach Lagerung für 2 Monate bei 30°C (t= 2 Monate). Der Nullwert (t=0) für diese fibrinogenhaltige Lösung ist nicht gezeigt, da sie ein Trennergebnis vergleichbar mit Figur 1a zeigt.

Der Hauptpeak bei einer Retentionszeit von ca. 15-16 min entspricht Fibrinogen. Bei dem Peak mit einer Retentionszeit von ca. 24 min sowie Peaks mit höheren Retentionszeiten handelt es sich um die Abbaufragmente von Fibrinogen. Der Figur 1b ist deutlich zu entnehmen, dass durch Verwendung des hydrophoben Gels die Fläche unter den Peaks, die den Abbaufragmenten entsprechen, nach zweimonatiger Lagerung bei 30 °C gegenüber dem Kontrollmaterial ohne HIC-Reinigung, das ebenfalls 2 Monate bei 30°C gelagert wurde (Figur 1c), deutlich reduziert ist. Das geringere Auftreten von Abbaufragmenten ist als Beweis für die höhere Stabilität von Fibrinogen nach Reinigung über eine negative HIC zu werten.

**Tabelle 1**

| | | | **Anstieg Abbaufragmente (% von allen Peaks)** | |
|---|---|---|---|---|
| **Gelmaterial** | **Puffer** | **Ausbeute (%)** | δ **(≤ Peak 4) nach 2 Mo** | δ **(≤ Peak 4) nach 3 Mo** |
| **Kationenaustauscher** | | | | |
| Fractogel EMD SO₃⁻ 650 | 1 | 78 | 4,1 | 6,0 |
| Heparin Fractogel | 1 | 91 | 4,2 | 6,4 |

| **Hydrohobes Gel** | | | | |
|---|---|---|---|---|
| Fractogel TA 650 | 2 | 81 | 4,3 | 5,8 |
| Butyl Cellufine | 2 | 73 | 3,9 | 6,4 |
| Fractogel EMD Butyl 650 | 2 | 82 | 4,1 | 5,9 |
| Fractogel EMD Propyl 650 | 2 | 91 | 3,8 | 5,7 |
| Macro Prep Methyl | 2 | 92 | 3,9 | 6,5 |
| Macro Prep t Butyl | 2 | 94 | 3,3 | 5,6 |
| Butyl Sepharose 4 Fast Flow | 2 | 82 | 4,1 | 6,2 |
| Thiopropyl Sepharose 6B | 2 | 96 | 4,3 | 6,4 |
| Butyl-S-Sepharose 6 Fast Flow (Prototyp) | 2 | 88 | 3,8 | 5,9 |
| Octyl Sepharose CL 4B | 2 | 91 | 4,0 | 6,1 |
| Phenyl Sepharose High Performance | 2 | 77 | 4,3 | 6,3 |
| Phenylalanin Sepharose | 2 | 91 | 4,0 | 6,7 |
| Hexyl S-Sepharos 6 Fast Flow | 2 | 57 | 3,6 | 5,8 |
| Pyridyl S-Sepharose Fast Flow | 2 | 68 | 3,5 | 5,5 |

| **Farbstoffgele** | | | | |
|---|---|---|---|---|
| Blue Hyper D | 3 | 68 | 4,8 | 5,7 |
| Fractogel TSK AF Green | 3 | 60 | 6,1 | 9,3 |
| Blue Sepharose CL 6B | 3 | 85 | 3,0 | 5,1 |
| Red Sepharose CL 6B | 3 | 73 | 3,9 | 6,4 |
| **Ausgangsmaterial** | AM | - | 16,8 | >90 |
| Lysin-Sepharose 4B **(Vergleichssäule)** | 1 | 98 | 6,7 | 11,2 |

Die Tabelle 1 zeigt, dass der Anteil an Abbaufragmenten mit erhöhter Retentionszeit nach Aufreinigung des verwendeten Fibrinogens über eines der aufgeführten Gelmaterialien deutlich weniger ansteigt als bei der Kontrolle (Ausgangsmaterial). Die damit nachgewiesene erhöhte Stabilität von Fibrinogen gegenüber der Kontrolle (Ausgangsmaterial vor negativer Chromatographie) ist bereits nach 1 Monat deutlich festzustellen, erhöht sich dann aber mit längeren Lagerzeiten noch. Nach längeren Lagerungszeiten von 3 Monaten, tendenziell aber bereits nach 2 Monaten, ist auch teilweise eine Diskreminierung der verschiedenen guten Säulenmaterialien möglich. Selbst gegenüber der im Stand der Technik bekannten Affinitätschromatographie mittels Lysin-Sepharose 4B, die Plasminogen abreichert, sind noch Verbesserungen zu erreichen. Durchweg werden auch sehr gute Ausbeuteergebnisse, in der Regel über ca. 70%, erzielt. Das Beispiel verdeutlicht weiterhin, dass mit einer großen Anzahl unterschiedlicher Kationenaustauscher, hydrophober Gele und Farbstoffgele eine wesentliche Stabilisierung von Fibrinogen und somit Verminderung der Bildung von Abbaufragmenten erreicht werden kann, so dass davon auszugehen ist, dass sich generell mit Trägermaterialien aus der Gruppe der genannten chromatographischen Trennprinzipien verbesserte Stabilitätsergebnisse erzielen lassen.

### Beispiel 2:

In diesem Beispiel wurden weitere blaue Farbstoffgele getestet und zusätzlich wurden für den in Beispiel 1 verwendeten Kationenaustauscher Fractogel EMD SO₃⁻ 650 (M) und das hydrophobe Gel Phenyl Sepharose HP die Pufferbedingungen der mobilen Phasen variiert. Die verbesserte Stabilität von Fibrinogen wurde wieder über die verminderte Bildung von Abbaufragmenten im Laufe eines Lagerversuches ermittelt. Zusätzlich wurde überprüft, ob es zur Abreicherung fibrinogenabbauender Proteine bzw. deren inaktiver Vorstufen (Proenzyme) kommt.

Als Ausgangsmaterial wurde Fibrinogen verwendet, das zusätzlich zu der analogen Aufreinigung aus Beispiel 1 nach der Aufnahme in einem geeigneten wässrigen Lösungmittel (50 mM NaCl; 20 mM tri-Natriumcitrat, 0,05% NaN₃ pH 7,4, Puffer AM in Tabelle 2) und vorzugsweise nach Dialyse gegen das Lösungsmittel, noch über Lysin-Sepharose aufgereinigt wurde.

Verwendet wurden Chromatographie-Säulen (Säulenkörper Ø=0,7 cm, h=2,5 cm von Qiagen), die jede mit 1,0 ml des jeweiligen Gelmaterials gefüllt waren. Das Gelmaterial wurde mit den entsprechenden Puffern äquilibriert.

**Äquilibrierungspuffer:**

| | | | |
|---|---|---|---|
| 1a: | 50 mM NaCl, | 20 mM Na₃-Citrat, | 0,05% NaN₃ pH 7,5 |
| 1b: | 50 mM NaCl, | 20 mM Na₃-Citrat, | 0,05% NaN₃ pH 6,5 |
| 2a: | 50 mM NaCl, | 20 mM Na₃-Citrat, | 0,05% NaN₃ pH 6,5 |
| 2b: | 50 mM NaCl, | 20 mM Na₃-Citrat, | 0,05% NaN₃ pH 7,0 |
| 2c: | 50 mM NaCl, | 20 mM Na₃-Citrat , | 0,05% NaN₃ pH 7,5 |
| 2f: | 150 mM NaCl, | 20 mM Na₃-Citrat, | 0,05% NaN₃ pH 7,5 |
| 3a: | 50 mM NaCl, | 20 mM Na₃-Citrat, | 0,05% NaN₃ pH 6,5 |
| 3b: | 50 mM NaCl, | 20 mM Na₃-Citrat, | 0,05% NaN₃ pH 7,5 |
| 3c: | 500 mM NaCl, | 20 mM Na₃-Citrat, | 0,05% NaN₃ pH 7,5 |
| 3d: | 1000 mM NaCl, | 20 mM Na₃-Citrat, | 0,05% NaN₃ pH 7,5 |

Die fibrinogenhaltige Auftragslösung wurde gegebenenfalls durch NaCl Zugabe und Einstellung des pH-Wertes an die obigen Pufferbedingungen angeglichen und ein Lösungsvolumen entsprechend einer Proteinmenge von 150 oder 300 OD₂₈₀₋₃₂₀ pro ml Gel (siehe Tabelle 2) bei freier Tropfgeschwindigkeit aufgetragen. Die Säulen wurden mit jeweils 1ml des entsprechenden Puffers gewaschen. Die Waschlösung wurde mit dem jeweiligen Durchlauf vereinigt und gegen 50 mM NaCl, 20 mM Na₃-Citrat, 0.05% NaN₃ pH 7,4 über Nacht bei 4°C dialysiert. Die Lagerung erfolgte bei 30°C für unterschiedlich lange Lagerzeiten (0 - 2 Monate (Mo)).

Die Analyse mit Hilfe der SEC-HPLC erfolgte wie unter Beispiel 1 beschrieben.

Zusätzlich wurde mit Hilfe von ELISA-Messungen die Menge an Plasminogen, Faktor XI und Faktor XII bestimmt. Plasminogen wurde in einem Sandwich-ELISA zunächst an polyklonale Antikörper vom Kaninchen (IgG-Präparation von Dade Behring), die als Fängerantikörper auf einer Mikrotiterplatte immobilisiert waren, gebunden. Die Detektion erfolgte mit Hilfe der gleichen polyklonalen Antikörperpräparation, die jedoch mit Peroxidase markiert war. F XII wurde mit Hilfe des F XII ELISA Kits von Kordia Life Sciences (Netherlands) entsprechend den Herstellerangaben bestimmt. Ebenso wurde F XI mit dem F XI ELISA Kit von Kordia Life Sciences (Netherlands) entsprechend den Herstellerangaben quantifiziert.

Der Abreicherungsfaktor (AF) ist der Quotient aus der Menge des bestimmten Proteins (z.B. Plasminogen, F XI oder F XII) pro OD₂₈₀₋₃₂₀ im Ausgangsmaterial und der Menge des bestimmten Proteins pro OD₂₈₀₋₃₂₀ in der fibrinogenhaltigen Lösung nach der jeweiligen negativen Chromatographie.

Die Ergebnisse sind in Tabelle 2 wiedergegeben.

**Tabelle 2**

| **Gel-material** | **Puffer** | **Beladun g der Säule OD**₂₈₀₋₃₂₀ | **Ausbeute (%)** | δ **Peak 4 nach 1 Mo/30°C** | δ **Peak 4 nach 2 Mo/30°C** | **AF Plasminogen** | **AF F XII** | **AF F XI** |
|---|---|---|---|---|---|---|---|---|
| Blue Hyper D | 1a | 150 | 61 | 1,03 | 2,25 | 3,7 | 21,5 | 2,0 |
| Mimetic Blue SA P6XL | 1a | 150 | 94 | 1,18 | 2,68 | 1,6 | 1,3 | ≥ 2,2 |
| Blue Trisacryl Plus LS | 1a | 150 | 74 | 1,13 | 2,42 | 2,4 | 22,1 | ≥ 2,2 |
| Blue Sepharose 6FF | 1a | 150 | 74 | 1,13 | 2,47 | 4,0 | 9,5 | ≥ 2,2 |
| | 1b | 150 | 61 | 1,12 | 2,75 | 2,4 | 12,6 | ≥ 2,2 |
| Fractogel EMD SO₃⁻ 650 | 2a | 150 | 45 | 1,57 | 2,63 | 3,8 | 12,4 | ≥ 2,2 |
| | 2b | 150 | 56 | 1,51 | 2,64 | 3,2 | 15,1 | ≥ 2,2 |
| | 2c | 150 | 80 | 1,06 | 2,68 | 1,8 | 1,6 | ≥ 2,2 |
| | 2f | 150 | 94 | 1,58 | 3,52 | 2,1 | 1,2 | ≥2,2 |
| Phenyl Sepharose High | 3a | 150 | 87 | 1,32 | 3,03 | 2,4 | 1,8 | 1,3 |
| | 3b | 150 | 91 | 1,30 | 3,10 | 2,0 | 1,1 | 1,3 |
| | 3c | 150 | 76 | 1,17 | 2,81 | 1,7 | 0,9 | 1,7 |
| Performance | 3d | 150 | 66 | 1,09 | 2,69 | 1,8 | 0,9 | ≥ 2,2 |
| | 3b | 300 | 96 | 1,43 | 3,27 | 2,0 | 1,2 | 1,5 |
| Ausgangsmaterial | AM | - | - | 1,85 | 4,33 | - | - | - |

| | | | | | | | | |
|---|---|---|---|---|---|---|---|---|
| AF: Abreicherungsfaktor | | | | | | | | |

Wie aus Tabelle 2 hervorgeht, ist die Anwendung auch der weiteren getesteten blauen Farbstoffgele geeignet, das Entstehen von Abbaufragmenten zu vermindern, d.h. eine erhöhte Stabilität von Fibrinogen zu erzielen. Gleichzeitig wird beispielhaft nachgewiesen, dass durch die verwendeten negativen Chromatographien, bei entsprechender Wahl der Trennparameter, Plasminogen, F XII und F XI -wenn auch in unterschiedlichem Maßeabgereichert werden können.

Erwartungsgemäß zeigt die Effizienz des Kationenaustauschers (Fractogel EMD SO₃⁻ 650) eine Abhängigkeit von der mobilen Phase (Pufferzusammensetzung). Bei einem pH-Wert von etwa 6,5-7.5 der mobilen Phase ist eine gute Abreicherung von Plasminogen und F XII nachweisbar. Bei einer Erhöhung des pH-Wertes der Trennung nimmt die Stabilität des Fibrinogens bei Flüssiglagerung ab, ist aber noch deutlich besser, als bei dem Ausgangsmaterial. Da auf der anderen Seite die Ausbeute an Fibrinogen bei niedrigen pH Werten abnimmt, da die Wechselwirkungen von Fibrinogen mit den funktionellen Gruppen zunehmen und es zum Teil bereits an das Säulenmaterial adsorbiert, liegen im Falle des Kationentauschers Fractogel EMD SO₃⁻ 650 (M) die besten Bedingungen für die mobile Phase im pH-Bereich um 7,0. Eine gleichzeitige Erhöhung der Salzkonzentration verbessert zwar die Ausbeute an Fibrinogen, die Stabilität nimmt aber gleichzeitig etwas ab. Im Falle von Fractogel EMD SO₃⁻ 650 (M) würde man also bevorzugt bei einer Salzkonzentration im Bereich von etwa 50 mM NaCl bleiben und zur Verbesserung der Ausbeute z.B. die Auftragsmenge pro ml Gel erhöhen. Ähnlich lassen sich optimale Zusammensetzungen der mobilen Phasen auch für andere Kationenaustauscher austesten.

Auch hydrophobe Gele zeigen eine Abhängigkeit von der mobilen Phase, insbesondere von der Salzkonzentration des Puffers. Bei der Phenyl Sepharose HP etwa ist über einen weiten Bereich an NaCl beispielsweise eine Abreicherung von Plasminogen sowie eine erhöhte Stabilität des Fibrinogen, bei einer guten Ausbeute an Fibrinogen, zu erreichen. Alle gewählten Bedingungen führen zu einer erhöhten Stabilität im Vergleich zum Ausgangsmaterial, so dass grundsätzlich mehrere Bedingungen vorteilhaft einsetzbar sind. Andere hydrophobe Gele und/oder andere Puffer-Bedingungen lassen sich auf ähnliche Weise optimieren.

Aus der Tabelle ist weiterhin ersichtlich, dass die unterschiedlichen Gele in diesem Beispiel zu einer unterschiedlich starken Abreicherung von Plasminogen, F XII und F XI beitragen. Eine besonders effiziente Abreicherung von FXII war beispielsweise durch die blauen Farbstoffgele Blue Hyper D und Blue Trisacryl Plus LS, Blue Sepharose 6FF oder Kationentauscher wie Fractogel EMD SO₃⁻ 650 (M) zu erreichen.

### Beispiel 3:

In diesem Beispiel wurden weitere Farbstoff-, Kationentauscher- und hydrophoben Gele bzw. Bedingungen für die negative Chromatographie getestet.

Das Ausgangsmaterial wurde nach demselben Reinigungsschema gewonnen wie in Beispiel 2 beschrieben. Die Säulen wurden, wie bereits in Beispiel 2 beschrieben, mit dem jeweiligen nachfolgend aufgeführten Puffer äquilibriert.

| | | | |
|---|---|---|---|
| 1: | 50 mM NaCl, | 20 mM Na₃-Citrat, | 0,05% NaN₃ pH 7,5 |
| 2: | 50 mM NaCl, | 20 mM Na₃-Citrat, | 0,05% NaN₃ pH 7,0 |
| 3a: | 1000 mM NaCl, | 20 mM Na₃-Citrat, | 0,05% NaN₃ pH 7,5 |
| 3b: | 2000 mM NaCl, | 20 mM Na₃-Citrat, | 0,05% NaN₃ pH 7,5 |

Das Ausgangsmaterial wurde gegen die entsprechenden Puffer dialysiert. Die Chromatographiesäulen wurden jeweils mit einer OD₂₈₀₋₃₂₀ von 150 oder 300 (entsprechend 15 ml oder 30 ml) pro ml Gel bei freier Tropfgeschwindigkeit beladen. Die ersten 0,5 ml der Säulendurchläufe wurden verworfen. Die Säulen wurden mit 1 ml des jeweiligen Puffers gewaschen. Die Waschlösung wurde mit dem jeweiligen Durchlauf vereinigt und gegen 50 mM NaCl, 20 mM Na₃-Citrat, 0.05% NaN₃ pH 7,4 über Nacht bei 4°C dialysiert. Die Lagerung erfolgte bei 30°C für eine Lagerzeit von 2 Monaten.

Die Analyse mit Hilfe der SEC-HPLC erfolgte wie unter Beispiel 1 beschrieben und die Bestimmung der Abreicherunsfaktoren (AF) für Plasminogen, F XII und F XI erfolgte wie unter Beispiel 2 beschrieben.

**Tabelle 3:**

| **Gel-material** | **Puffer** | **Beladung der Säule OD** _{280- 320} | **Gelvolumen (ml)** | **Ausbeute (%)** | **AF Plasminogen** | **AF F XII** | **AF F XI** | δ **Peak 4 nach 2Mo/ 30°C** |
|---|---|---|---|---|---|---|---|---|
| Blue Uniflow | 1 | 150 | 1,0 | 85 | 1,5 | 15,4 | 2,7 | 1,63 |
| Blue Trisacryl Plus LS | 1 | 150 | 1,0 | 77 | 4,8 | >45,3 | > 4,3 | 1,55 |
| Fractogel EMD SO₃⁻ 650 (M) | 2 | 150 | 1,0 | 72 | 1,1 | 29,2 | > 4,3 | 1,64 |
| | 2 | 300 | 1,0 | 82 | 1,5 | 7,7 | > 4,3 | 2,02 |
| Macro Prep t Butyl HIC Support | 3a | 150 | 1,0 | 100 | 0,8 | 1,1 | 1,4 | 2,11 |
| | 3b | 150 | 1,0 | 89 | 1,1 | 1,3 | 1,6 | 2,08 |
| Fractogel EMD Butyl 650 (S) | 3a | 150 | 1,0 | 91 | 1,4 | 1,5 | 1,8 | 1,63 |
| Ausgangsmaterial | AM | - | - | - | - | - | - | 2,32 |

Tabelle 3 zeigt, dass auch die weiteren getesteten Gele bzw. Bedingungen geeignet sind, die Entstehung von Abbaufragmenten zu vermindern. Eine Erhöhung der Auftragsmenge (Beladung der Säule), führt zu einer verbesserten Ausbeute.

### Beispiel 4:

Einige Gelmaterialien, die sich aufgrund der Beispiele 1 bis 3 als geeignet erwiesen, wurden auf ihre Tauglichkeit in größerem Maßstab getestet. Dazu wurden Chromatographiesäulen mit ca. 500 ml Gelvolumen verwendet und die fibrinogenhaltige Lösung durch die Säulen gepumpt.

Für die Gewinnung einer fibrinogenhaltigen Lösung wurde Kryopräzipitat entsprechend EP 0 103 196 bis zur pasteurisierten Fibrinogenlösung aufgearbeitet.

Die nun pasteurisierte Fibrinogenlösung wurde mit dem dreifachen Volumen Verdünnungslösung (3,5 g/l NaCl; 5,88 g/l tri-Natriumcitrat-dihydrat in Wasser, pH 7,5) versetzt. Pro Liter verdünnter Lösung wurden 90 g Glycin unter Rühren zugesetzt . Das entstandene Präzipitat wurde mittels Zentrifugation oder Filtration abgetrennt und verworfen.

Der Überstand wurde optional auf 200 mM L-Lys x HCl oder EACA durch Zugabe von festem L-Lys x HCl oder EACA gebracht. Pro Liter wurden weitere 75 g Glycin zugesetzt. Das fibrinogenreiche Präzipitat wurde mittels Zentrifugation gewonnen und bis zur weiteren Verarbeitung bei -25°C gelagert.

Für die weitere Reinigung und Abreicherung von Plasminogenspuren wurde das fibrinogenreiche Präzipitat gelöst und vorzugsweise nach Dialyse gegen Pufferlösung (20 mM tri-Natriumcitrat, 50 mM NaCl pH 7,4, optional enthaltend 0,05 % NaN₃ als Konservierungsmittel), über eine Chromatographiesäule mit einer Matrix, die als Liganden L-Lysyl Reste trägt, gepumpt. Der Durchlauf wurde für die nachfolgenden Schritte weiter verwendet.

Zur Abtrennung weiterer, die Stabilität von Fibrinogen beinflussender Kontaminanten wurde die fibrinogenhaltige Lösung über verschiedene zweite Chromatographiesäulen gepumpt, gegebenenfalls mit vorheriger Änderung der Pufferzusammensetzung:
A: Die fibrinogenhaltige Lösung wurde direkt über eine Säule (Ø 6 cm, Volumen ca. 735 ml) mit blauem Farbstoffgel, das über eine Matrix verfügt, die als Liganden einen derivatisierten Anthrachinon Farbstoff trägt (wie z.B. Blue Agarose von Prometic) gepumpt und der Durchlauf gesammelt. Die Säule wurde mit 1 Säulenvolumen (SV) Pufferlösung (20 mM tri-Natriumcitrat, 50 mM NaCl pH 7,4, optional enthaltend 0,05 % NaN₃ als Konservierungsmittel) nachgewaschen.
B: Die fibrinogenhaltige Lösung wurde durch Zusatz von 0,1 M HCl auf einen pH von 6,7 eingestellt und über eine Säule (Ø 6 cm, Volumen ca. 500 ml oder Ø 6 cm, Volumen ca. 147 ml) mit einer Matrix, die als Liganden SO₃⁻-Gruppen trägt (Fractogel EMD SO₃⁻ 650 (M)) gepumpt. Der Durchlauf wurde gesammelt. Die Säule wurde mit 1 SV Pufferlösung (20 mM tri-Natriumcitrat, 50 mM NaCl pH 6,5, optional enthaltend 0,05 % NaN₃ als Konservierungsmittel) nachgewaschen.
C: Die fibrinogenhaltige Lösung wurde durch Zusatz von kristallinem NaCl auf eine Endkonzentration von 1 M NaCl gebracht und über eine Säule (Ø 6 cm, Volumen ca. 500 ml) mit einer hydrophoben Matrix, die als Liganden Phenylgruppen trägt (Phenyl-Sepharose HP) gepumpt und der Durchlauf gesammelt. Die Säule wurde mit 1 SV Pufferlösung (20 mM tri-Natriumcitrat, 1 M NaCl pH 7,4, optional enthaltend 0,05 % NaN₃ als Konservierungsmittel) nachgewaschen.
D: Die fibrinogenhaltige Lösung wurde durch Zusatz von kristallinem NaCl auf eine Endkonzentration von 1 M NaCl gebracht und über eine Säule (Ø 7 cm, Volumen ca. 577 ml) mit einer hydrophoben Matrix, die als Liganden Butylgruppen trägt (Macro Prep t Butyl HIC Resin) gepumpt und der Durchlauf gesammelt. Die Säule wurde mit 1 SV Pufferlösung (20 mM tri-Natriumcitrat-dihydrat, 1 M NaCl pH 7,4, optional enthaltend 0,05 % NaN₃ als Konservierungsmittel) nachgewaschen.

Zur Herstellung von Fibrinogen-Zubereitungen wurden die fibrinogenhaltigen Durchläufe mit den jeweiligen Waschlösungen vereinigt und zunächst mittels geeigneter Ultrafiltrationsverfahren, je nach Verwendung, auf eine Proteinkonzentration von ca. OD₂₈₀₋₃₂₀ₙₘ = 2 - 200, vorzugsweise ca. 20 - 160, gebracht und anschließend gegen Lösungen dialysiert, die die folgenden Formulierungsbestandteile enthielten: NaCl, Na₃-Citrat x 2 H₂O, L-Arg x HCl, optional CaCl₂.

Nach abschließender Ankonzentrierung und Sterilfiltration erhielt man Fibrinogen-Zubereitungen, die mit Hilfe der SEC-HPLC (siehe Beispiel 1) nach 1 Monat Lagerung bei 30 °C auf den Gehalt an Fibrinogen-Abbaufragmenten getestet wurden (siehe Tabelle 4).

**Tabelle 4:**

| **Gelmaterial** | **Puffer** | δ **(**≤ **Peak 4) nach 1 Mo/ 30°C** |
|---|---|---|
| Blue Agarose | 20 mM Na₃Citrat, 100 mM NaCl, 50 g/l L-ArgxHCl, 2,5 mM CaCl₂ pH 7,2 | 0,9 |
| Fractogel EMD SO₃⁻ 650 (M) | 20 mM Na₃ Citrat, 100 mM NaCl, 50 g/l L-ArgxHCl, 2,5 mM CaCl₂ pH 7,2 | 1,0 |
| Phenyl Sepharose High Performance | 20 mM Na₃ Citrat, 100 mM NaCl, 50 g/l L-ArgxHCl, 2,5 mM CaCl₂ pH 7,2 | 1,2 |
| Macroprep-t Butyl HIC Support | 20 mM Na₃Citrat, 100 mM NaCl, 50 g/l L-ArgxHCl, 2,5 mM CaCl₂ pH 7,2 | 1,1 |
| Ohne zusätzliche negative Chromatographie | 20 mM Na₃ Citrat, 100 mM NaCl, 50 g/l L-ArgxHCl, 2,5 mM CaCl₂ pH 7,2 | 2,6 |

Die Ergebnisse zeigen, dass auch in größerem Maßstab durch Verwendung negativer Chromatographien die Differenz der nach akzelerierter Lagerung für 1 Monat entstehenden Abbaufragmente (δ (≤ Peak 4)) auf unter 2% reduziert werden kann und die Stabilität des Fibrinogen in Lösung somit bei einer Lagerung bei 30 °C deutlich erhöht ist.

### Beispiel 5:

In diesem Beispiel wurden zwei negative Adsorptionen im Batch-Format unter Verwendung eines Farbstoffgels und eines hydrophoben Gels kombiniert.

Für die Gewinnung einer fibrinogenhaltigen Lösung wurde Kryopräzipitat als Ausgangsmaterial wie in EP 0 103 196 beschrieben zweimal mit Al(OH)₃ Suspension adsorbiert.

Zur Reduktion/Entfernung kontaminierender Proteine wie insbesondere fibrinogenabbauender Proteine, wurden weitere Adsorptionen im Batch-Format durchgeführt. Dazu wurde die Blue Sepharose 6 FF verwendet, ein Gelmaterial, das als Liganden einen derivatisierten Anthrachinon Farbstoff trägt. Je 10 g fibrinogenhaltiger Lösung wurden 0,5 g nutschenfeuchtes Gel zugesetzt. (Nachrührzeit: 90 min) Anschließend wurde das Farbstoffgel durch Zentrifugation (20 min bei 25°C und 1500 g) entfernt.

Der fibrinogenhaltige Überstand wurde einer weiteren negativen Adsorption unterworfen. Dazu wurde Phenyl Sepharose HP verwendet. Das Gelmaterial wurde ebenfalls im Verhältnis 0,5 g pro 10 g fibrinogenhaltiger Lösung eingesetzt (Nachrührzeit: 90 min). Im Anschluß an die Adsorption wurde das Gelmaterial mit den gebundenen kontaminierenden Proteinen durch Zentrifugation entfernt.

Die anschließende Pasteurisierung und Glycin-Fällung wurde entsprechend EP 0 103 196 durchgeführt mit der Ausnahme, dass die Fällung in Anwesenheit von 200 mM Lys erfolgte.

Für die weitere Reinigung und Plasminogenentfernung wurde das fibrinogenreiche Präzipitat zunächst in einem geeigneten wässrigen Lösungmittel gelöst, filtriert und vorzugsweise nach Dialyse gegen Pufferlösung (20 mM tri-Natriumcitrat-dihydrat, 50 mM NaCl, pH 7,4, optional enthaltend 0,05 % NaN₃ als Konservierungsmittel) und Einstellung einer optische Dichte von ca. 10 über eine Chromatographiesäule mit einem Gelmaterial, das als Liganden L-Lysyl Reste trägt, gepumpt.

Zur Herstellung von Fibrinogen-Zubereitungen wurde die fibrinogenhaltige Lösung zunächst mittels geeigneter Ultrafiltrationsverfahren je nach Verwendung auf eine

Proteinkonzentration von ca. OD₂₈₀₋₃₂₀ₙₘ = 2 - 200, vorzugsweise ca. 20 - 160, gebracht und anschließend gegen Lösungen, die die im Beispiel 4 genannten Formulierungsbestandteile enthielten, dialysiert.

Nach Sterilfiltration erhielt man Fibrinogen-Zubereitungen, die entsprechend Beispiel 1 mit Hilfe der SEC-HPLC auf Stabilität getestet wurde. Danach ergab sich nach Lagerung von 1 Monat bei 30°C und nach Abzug des Nullwertes ein Anteil an Abbaufragmenten von <1,0 %, gemessen anhand von Peak 4 und kleineren Peaks, also deutlich weniger als bei entsprechenden Kotrollaufarbeitungen.

Mit diesem Beispiel konnte gezeigt werden, dass auch negative Adsorptionen im Batch-Format anwendbar sind, um eine möglichst hohe Stabilität des Fibrinogen in Lösung zu erreichen. Zudem wurde gezeigt, dass mehrere negative Adsorptionen kombiniert werden können. Weiterhin wird ersichtlich, dass sich Verfahrensschritte mit negativen Adsorptionen und/oder negativen Chromatographien sinnvoll an unterschiedlichen Stellen im Verfahren zur Reinigung von Fibrinogen integrieren lassen. So wird in diesem Beispiel die negative Adsorption, im Gegensatz zu den vorherigen Beispielen, sehr früh im Reinigungsverfahren, direkt nach der Aluminiumhydroxid Behandlung angewendet. Pasteurisierung, Präzipitation mit Glycin und Entfernung weiteren Plasminogens durch Lysin-Sepharose erfolgten erst im Anschluß.

### Beispiel 6:

In diesem Beispiel wurde untersucht, inwieweit mit Hilfe von Ultrafiltration, durch Auswahl geeigneter Porengrößen, fibrinogenabbauende Proteine abgereichert werden können.

Für die Gewinnung einer fibrinogenhaltigen Lösung wurde bis einschließlich der Herstellung eines pasteurisierten, gefällten Präzipitats entsprechend Beispiel 1 vorgegangen.

Für die weitere Reinigung und Abreicherung fibrinogenabbauender Proteine wurde das fibrinogenreiche Präzipitat zunächst in einem geeigneten wässrigen Lösungmittel gelöst und unter Einsatz von Ultrafiltrationsmembranen mit einem cut-off von 300 kDa intensiv gegen Pufferlösung (20 mM tri-Natriumcitrat-dihydrat, 50 mM NaCl pH 7,4, optional enthaltend 0,05 % NaN₃ als Konservierungsmittel) diafiltriert.

Anschließend wurde die Lösung zur Plasminogenentfernung über eine Chromatographiesäule mit einem Gelmaterial, das als Liganden L-Lysyl Reste trägt, gepumpt.

Zur Herstellung von Fibrinogen-Zubereitungen wurde die fibrinogenhaltige Lösung zunächst mittels geeigneter Ultrafiltrationsverfahren und -Membranen (cut off = 300 kDa) je nach Verwendung auf eine Proteinkonzentration von ca. OD₂₈₀₋₃₂₀ₙₘ = 2 - 200, vorzugsweise ca. 20 - 160, gebracht und anschließend gegen Lösungen, die geeignete Formulierungsbestandteile enthielten, dialysiert.

Nach abschließender Ankonzentrierung und Sterilfiltration erhielt man Fibrinogen-Zubereitungen, die mit Hilfe der SEC-HPLC (siehe Beispiel 1) vor Beginn der Lagerung und nach 1 Monat Lagerung bei 30 °C auf den Gehalt an Fibrinogen-Abbaufragmenten getestet wurden. Danach ergab sich, nach Abzug des Nullwertes, ein verringerter Anteil an Abbaufragmenten gegenüber einer Kontrolle mit herkömmlicher Ultrafiltration.

Damit konnte gezeigt werden, dass durch Ultrafiltration mit einem cut off von 300 kDa weitere fibrinogenabbauende Proteine abgereichert werden können und ein stabileres Fibrinogenkonzentrat herstellbar ist.

### Beispiel 7:

Wie in Beispiel 1 beschrieben wurde ein Fibrinogenpräzipitat hergestellt und zusätzlich mittels negativer Chromatographie an Lys-Sepharose gereinigt. Nach Aufstockung auf 1 Mol Natriumchlorid pro Liter Fibrinogenlösung wurde diese auf eine mit Butyl-Sepharose gefüllte Chromatographiesäule gegeben und mit Puffer nachgespült. Der Durchlauf der Säule wurde ankonzentriert, dialysiert und auf den Gehalt an t-PA, Plasminogen und F XI getestet. Als Kontrolle wurde eine Vergleichsaufarbeitung durchgeführt, bei der die negative Chromatographie an Butyl-Sepharose nicht durchgeführt wurde. Als Ergebnis konnte gezeigt werden, dass durch die zusätzliche HIC die Konzentration an t-PA, Plasminogen und F XI deutlich reduziert wurden und dass die Stabilität nach Lagerung bei 30°C erhöht ist (der Anteil an Fibrinogenfragmenten nach 1 Monat bei 30°C war um ca. 20% geringer, wenn die HIC durchgeführt wurde).

| | t-PA (ng/OD280-320) | Plasminogen (ng/OD280-320) | F XI (ng/OD280-320) | Fibrinogenfragmente nach 1 Monat bei 30°C (Aufarbeitung ohne HIC = 100%) |
|---|---|---|---|---|
| Aufarbeitung ohne HIC | 0,046 | 2,47 | 0,069 | 100 % |
| Aufarbeitung inkl. HIC | 0,003 | 2,04 | 0,038 | 79 % |

### Beispiel 8:

Wie in Beispiel 1 beschrieben wird ein Fibrinogenpräzipitat hergestellt und mittels negativer Chromatographie an Lys-Sepharose gereinigt. Die weitere Reinigung erfolgt mittels Farbstoffsäule, HIC und/oder Kationentauscher. Die Fibrinogenlösung wird durch Diafiltration und Ultrafiltration in folgende Formulierungspuffer überführt und nach Sterilfiltration akzeleriert bei 30°C gelagert:
1. 20 mM Na₃-Citrat, 100 mM NaCl, 50 g/l L-Arg x HCl pH 7,2
2. 20 mM Na₃-Citrat, 100 mM NaCl, 70 g/l L-Arg x HCl pH 7,2
3. 20 mM Na₃-Citrat, 100 mM NaCl, 100 g/l L-Arg x HCl pH 7,2
4. 20 mM Na₃-Citrat, 100 mM NaCl, 50 g/l L-Arg x HCl, 2,5 mM CaCl₂ pH 7,2
5. 20 mM Na₃-Citrat, 100 mM NaCl, 70 g/l L-Arg x HCl, 2,5 mM CaCl₂ pH 7,2
6. 20 mM Na₃-Citrat, 100 mM NaCl, 100 g/l L-Arg x HCl, 2,5 mM CaCl₂ pH 7,2
7. 4 mM Na₃-Citrat, 100 mM NaCl, 50 g/l L-Arg x HCl, 0,5 mM CaCl₂ pH 7,2
8. 12 mM Na₃-Citrat, 100 mM NaCl, 50 g/l L-Arg x HCl, 1,5 mM CaCl₂ pH 7,2
9. 20 mM Na₃-Citrat, 100 mM NaCl, 50 g/l L-Arg x HCl, 2,5 mM CaCl₂ pH 7,2
10. 20 mM Na₃-Citrat, 200 mM NaCl, 50 g/l L-Arg x HCl, 2,5 mM CaCl₂ pH 7,2
11. 20 mM Na₃-Citrat, 100 mM NaCl, 70 g/l L-Arg x HCl, 2,5 mM CaCl₂ pH 6,8
12. 20 mM Na₃-Citrat, 100 mM NaCl, 60 g/l L-Arg x HCl, 1% L-His, 2,5 mM CaCl₂ pH 7,2
13. 20 mM Na₃-Citrat, 100 mM NaCl, 60 g/l L-Arg x HCl, 2% L-His, 2,5 mM CaCl₂ pH 7,2
14. 4 mM Na₃-Citrat, 100 mM NaCl, 60 g/l L-Arg x HCl, 0,5 mM CaCl₂ pH 7,2
15. 20 mM Na₃-Citrat, 100 mM NaCl, 60 g/l L-Arg x HCl, 30 mM Aminobenzoesäure, 2,5 mM CaCl₂ pH 7,2
16. 4 mM Na₃-Citrat, 100 mM NaCl, 60 g/l L-Arg x HCl pH 7,2
17. 4 mM Na₃-Citrat, 100 mM NaCl, 60 g/l L-Arg x HCl, 2% L-His pH 7,2
18. 4 mM Na₃-Citrat, 100 mM NaCl, 60 g/l L-Arg x HCl, 2% L-His pH 6,4
19. 4 mM Na₃-Citrat, 100 mM NaCl, 60 g/l L-Arg x HCl, 2% L-His, 0,5 mM CaCl₂ pH 7,2
20. 20 mM Na₃-Citrat, 100 mM NaCl, 60 g/l L-Arg x HCl, 2,5 mM CaCl₂ pH 7,2

Die Stabilität der aufgeführten Formulierungen ist sehr gut und eine Fragmentbildung wird nur in sehr geringem Maße beobachtet (es entstehen weniger als 2% Fragmente (δ (≤ Peak 4)) während einer einmonatigen Lagerung bei 30°C). Die Lösungen sind geignet, als Bestandteil eines im flüssigen Zustand lagerfähigen Fibrinklebers bestehend aus zwei oder mehr Komponenten zu dienen.

## Patentansprüche

1. Verfahren zur Reinigung von Fibrinogen-haltigen Lösungen, **dadurch gekennzeichnet, dass** es einen oder mehrere Verfahrensschritte enthält, bei denen ein oder mehrere kontaminierende Proteine durch eine oder mehrere negative Chromatographien und/oder eine oder mehrere negative Adsorptionen unter Verwendung von Farbstoffgel abgereichert werden, wobei die negative Chromatographie und/oder die negative Adsorption bei einem pH-Wert zwischen 5,5 und 9 durchgeführt wird.

2. Verfahren nach Anspruch 1, **dadurch gekennzeichnet, dass** als Farbstoffgel ein blaues Farbstoffgel verwendet wird.

3. Verfahren nach Anspruch 1, **dadurch gekennzeichnet, dass** als Farbstoffgel ein rotes oder grünes Farbstoffgel verwendet wird.

4. Verfahren nach einem der Ansprüche 1 bis 3, **dadurch gekennzeichnet, dass** es sich bei dem Farbstoffgel um Blue Hyper D, Mimetic Blue Agarose, Mimetic Blue SA P6XL, Mimetic Blue 1 P6XL, Blue Trisacryl Plus LS, Blue Uniflow, Blue Sepharose 6FF, Blue Sepharose CL 6B, Red Sepharose CL 6B, Fractogel TSK AF Green und/oder Matrex gel Green A handelt.

5. Verfahren nach einem der Ansprüche 1 bis 4, **dadurch gekennzeichnet, dass** die Ausbeute von Fibrinogen im Durchlauf der negativen Chromatographie oder im Überstand der negativen Adsorption ≥ 50%, bevorzugt ≥ 70% ist.

6. Verfahren nach einem der Ansprüche 1 bis 5, **dadurch gekennzeichnet, dass** der Verfahrensschritt, der die negative Adsorption oder Chromatographie beinhaltet, in Anwesenheit von Substanzen, die die Bindung von Plasminogen an Fibrinogen schwächen, durchgeführt wird.

7. Verfahren nach einen der Ansprüche 1 bis 6, **dadurch gekennzeichnet, dass** als Ausgangsmaterial eine aus Blut gewonnene Mischung, Milch von transgenen Tieren oder ein Fermentationsüberstand oder eine daraus hergstellte Fraktion verwendet wird.

8. Verfahren nach Anspruch 7, **dadurch gekennzeichnet, dass** als Ausgangsmaterial humanes Plasma, eine Plasmafraktion oder Kryopräzipitat verwendet wird.

9. Verfahren nach einem der Ansprüche 1-8, **dadurch gekennzeichnet, dass** ein oder mehrere Verfahrensschritte enthalten sind, bei denen Fibrinogen präzipitiert wird.

10. Verfahren nach Anspruch 9, **dadurch gekennzeichnet, dass** ein oder mehrere Präzipitationen mit Glycin oder anderen Aminosäuren enthalten sind.

11. Verfahren nach einem der Ansprüche 1-10, **dadurch gekennzeichnet, dass** ein oder mehrere Verfahrensschritte enthalten sind, bei denen Plasminogen über Gelmaterial mit Lysin oder Lysin-Analoga als funktionelle Gruppe entfernt wird.

12. Verfahren nach einem der Ansprüche 1-11, **dadurch gekennzeichnet, dass** ein oder mehrere Verfahrensschritte zur Abreicherung und/oder Entfernung infektiöser Partikel enthalten sind.

13. Verfahren nach einem der Ansprüche 1-12, bei dem folgende Verfahrensschritte kombiniert werden: Herstellung einer Plasmafraktion, Adsorption an Aluminiumhydroxid, Inaktivierung infektiöser Partikel wie beispielsweise Viren, Präzipitation, weitere Reinigungs- und/oder Inaktivierungsschritte, negative Chromatographie und/oder negative Adsorption, Ultrafiltration, Sterilfiltration.

## Claims

1. Process for purifying fibrinogen-containing solutions, **characterized in that** it comprises one or more process steps in which one or more contaminating proteins are depleted by one or more negative chromatographies and/or one or more negative adsorptions using dye gel, wherein the negative chromatography and/or the negative adsorption is carried out at a pH between 5.5 and 9.

2. Process according to Claim 1, **characterized in that** a blue dye gel is used as dye gel.

3. Process according to Claim 1, **characterized in that** a red or green dye gel is used as dye gel.

4. Process according to any of Claims 1 to 3, **characterized in that** the dye gel is Blue Hyper D, Mimetic Blue Agarose, Mimetic Blue SA P6XL, Mimetic Blue 1 P6XL, Blue Trisacryl Plus LS, Blue Uniflow, Blue Sepharose 6FF, Blue Sepharose CL 6B, Red Sepharose CL 6B, Fractogel TSK AF Green and/or Matrex gel Green A.

5. Process according to any of Claims 1 to 4, **characterized in that** the yield of fibrinogen in the flow-through of the negative chromatography or in the supernatant of the negative adsorption is ≥ 50%, preferably ≥ 70%.

6. Process according to any of Claims 1 to 5, **characterized in that** the process step which includes the negative adsorption or chromatography is carried out in the presence of substances which weaken the binding of plasminogen to fibrinogen.

7. Process according to any of Claims 1 to 6, **characterized in that** a mixture obtained from blood, milk from transgenic animals or a fermentation supernatant or a fraction produced therefrom is used as starting material.

8. Process according to Claim 7, **characterized in that** human plasma, a plasma fraction or cryoprecipitate is used as starting material.

9. Process according to any of Claims 1 - 8, **characterized in that** it comprises one or more process steps in which fibrinogen is precipitated.

10. Process according to Claim 9, **characterized in that** it comprises one or more precipitations with glycine or other amino acids.

11. Process according to any of Claims 1 - 10, **characterized in that** it comprises one or more process steps in which plasminogen is removed on gel material with lysine or lysine analogs as functional group.

12. Process according to any of Claims 1 - 11, **characterized in that** it comprises one or more process steps for depletion and/or removal of infectious particles.

13. Process according to any of Claims 1 - 12, in which the following process steps are combined: preparation of a plasma fraction, adsorption onto aluminum hydroxide, inactivation of infectious particles such as, for example, viruses, precipitation, further purification and/or inactivation steps, negative chromatography and/or negative adsorption, ultrafiltration, sterilizing filtration.

## Revendications

1. Procédé de purification de solutions contenant du fibrinogène, **caractérisé en ce qu'**il contient une ou plusieurs étapes de procédé dans lesquelles une ou plusieurs protéines de contamination sont appauvries par une ou plusieurs chromatographies négatives et/ou une ou plusieurs adsorptions négatives, en utilisant un gel de colorant, où la chromatographie négative et/ou l'adsorption négative sont réalisées à un pH entre 5,5 et 9.

2. Procédé selon la revendication 1, **caractérisé en ce que** l'on utilise comme gel de colorant un gel de colorant bleu.

3. Procédé selon la revendication 1, **caractérisé en ce que** l'on utilise comme gel de colorant un gel de colorant rouge ou vert.

4. Procédé selon l'une quelconque des revendications 1 à 3, **caractérisé en ce que** le gel de colorant est le Blue Hyper D, le Mimetic Blue Agarose, le Mimetic Blue SA P6XL, le Mimetic Blue 1 P6XL, le Blue Trisacryl Plus LS, le Blue Uniflow, le Blue Sepharose 6FF, le Blue Sepharose CL 6B, le Red Sepharose CL 6B, le Fractogel TSK AF Green et/ou le Matrex gel Green A.

5. Procédé selon l'une quelconque des revendications 1 à 4, **caractérisé en ce que** le rendement de fibrinogène dans la partie éluée de la chromatographie négative ou dans la phase surnageante de l'adsorption négative est ≥ 50%, de préférence ≥ 70%.

6. Procédé selon l'une quelconque des revendications 1 à 5, **caractérisé en ce que** l'étape de procédé qui comporte l'adsorption ou la chromatographie négative est réalisée en présence de substances qui affaiblissent la liaison de plasminogène sur le fibrinogène.

7. Procédé selon l'une quelconque des revendications 1 à 6, **caractérisé en ce qu'**on utilise comme matériau de départ un mélange obtenu à partir du sang, du lait d'animaux transgéniques ou une phase surnageante d'une fermentation ou une fraction préparée à partir de ceux-ci.

8. Procédé selon la revendication 7, **caractérisé en ce qu'**on utilise comme matériau de départ du plasma humain, une fraction du plasma ou un cryoprécipité.

9. Procédé selon l'une quelconque des revendications 1 à 8, **caractérisé en ce qu'**il comprend une ou plusieurs étapes de procédé, dans lesquelles le fibrinogène est précipité.

10. Procédé selon la revendication 9, **caractérisé en ce qu'**il comprend une ou plusieurs précipitations avec de la glycine ou d'autres acides aminés.

11. Procédé selon l'une quelconque des revendications 1 à 10, **caractérisé en ce qu'**il comprend une ou plusieurs étapes de procédé, dans lesquelles le plasminogène est éliminé via le matériau de gel avec de la lysine ou des analogues de lysine comme groupe fonctionnel.

12. Procédé selon l'une quelconque des revendications 1 à 11, **caractérisé en ce qu'**il comprend une ou plusieurs étapes de procédé pour l'appauvrissement et/ou l'élimination de particules infectieuses.

13. Procédé selon l'une quelconque des revendications 1 à 12, dans lequel les étapes de procédé suivantes sont combinées : préparation d'une fraction de plasma, adsorption sur de l'hydroxyde d'aluminium, inactivation de particules infectieuses, telles que par exemple des virus, précipitation, autres étapes de purification et/ou d'inactivation, chromatographie négative et/ou adsorption négative, ultrafiltration, filtration stérile.
